(19) Europäisches Patentamt
European Patent Office
Office européen des brevets

(11) EP 4 438 586 A1

(12) # EUROPÄISCHE PATENTANMELDUNG

(43) Veröffentlichungstag:
**02.10.2024 Patentblatt 2024/40**

(21) Anmeldenummer: **23165364.3**

(22) Anmeldetag: **30.03.2023**

(51) Internationale Patentklassifikation (IPC):
***C07C 37/20*** *(2006.01)* ***C07C 39/16*** *(2006.01)*
***C07C 29/151*** *(2006.01)* ***C07C 1/20*** *(2006.01)*
***C07C 11/06*** *(2006.01)* ***C07C 41/09*** *(2006.01)*
***C07C 43/04*** *(2006.01)* ***C07C 45/28*** *(2006.01)*
***C07C 49/08*** *(2006.01)*

(52) Gemeinsame Patentklassifikation (CPC):
(C-Sets verfügbar)
**C07C 29/1518; C07C 1/20; C07C 37/20; C07C 41/09; C07C 45/28** (Forts.)

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**KH MA MD TN**

(71) Anmelder: **Covestro Deutschland AG**
**51373 Leverkusen (DE)**

(72) Erfinder: **Die Erfindernennung liegt noch nicht vor**

(74) Vertreter: **Levpat**
**c/o Covestro AG**
**Gebäude K12**
**51365 Leverkusen (DE)**

(54) **NACHHALTIGE HERSTELLUNG VON BISPHENOL-A FÜR DIE PRODUKTION VON POLYCARBONAT**

(57) Die vorliegende Erfindung betrifft ein Herstellungsverfahren von Bisphenol-A für die Synthese von Polycarbonat gemäß Anspruch 1, sowie ein Verfahren zur Herstellung von Polycarbonat aus dem gemäß vorgenanntem Herstellungsverfahren erhaltenen Bisphenol-A gemäß Anspruch 14 und ein Mehrkomponenten-System zur Bisphenol-A Herstellung gemäß vorgenanntem Herstellungsverfahren. Darin wird das Bisphenol-A aus bereitgestelltem Aceton und bereitgestelltem Phenol hergestellt, wobei diese Rohstoffe Verfahrensprodukte eines speziellen Herstellungsverfahrens sein müssen, welche auf den Einsatz von $CO_2$, Methan aus biologischer Quelle, Kohlehydrate und/oder organischen, festförmigen Verbindungen als Ausgangsstoffe zurückgreifen.

Fig.1:

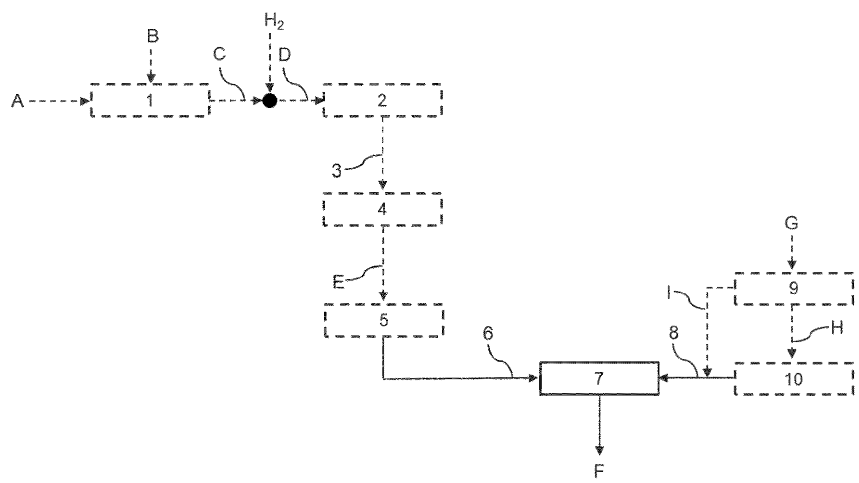


EP 4 438 586 A1

(52) Gemeinsame Patentklassifikation (CPC): (Forts.)

C-Sets
**C07C 1/20, C07C 11/06;**
**C07C 29/1518, C07C 31/04;**
**C07C 37/20, C07C 39/16;**
**C07C 41/09, C07C 43/043;**
**C07C 45/28, C07C 49/08**

## Beschreibung

Die vorliegende Erfindung betrifft ein Herstellungsverfahren von Bisphenol-A für die Synthese von Polycarbonat, sowie ein Verfahren zur Herstellung von Polycarbonat aus dem gemäß vorgenanntem Herstellungsverfahren erhaltenen Bisphenol-A und ein Mehrkomponenten-System zur Bisphenol-A Herstellung gemäß vorgenanntem Herstellungsverfahren.

Bisher basiert die industrielle Herstellung von Bisphenol-A überwiegend auf der Nutzung fossiler Rohstoffe, wie z.B. von aus Erdgas bereitgestelltem Synthesegas und erdölbasierten aromatischen Verbindungen. Die konsequente Nutzung von regenerativen Rohstoffen und/oder von Wertstoffen aus Abfällen für die Herstellung von Materialien wie Bisphenol-A ist ein Ziel für die Bereitstellung nachhaltiger Kunststoffe, wie Polycarbonat und Materialien daraus.

Weiterhin wird die Nutzung von Energie aus regenerativen Energiequellen für die industrielle Herstellung chemischer Rohstoffe, wie z.B. von Bisphenol-A, angestrebt.

In der Druckschrift US 2010/0152406 A1 wird beschrieben, dass sich Bisphenol-A aus erneuerbarem Phenol und erneuerbarem Aceton, i.e. Phenol und Aceton aus erneuerbaren Rohstoffen, herstellen lässt. Aceton soll demgemäß aus den erneuerbaren Rohstoffquellen Bio-Ethanol oder durch Fermentation hergestellt werden. Allerdings werden gemäß Lehre der besagten Druckschrift die darin für die Herstellung von erneuerbarem Aceton skizzierten Routen als nicht geeignet für eine Herstellung im industriellen Maßstab angesehen.

Es war somit eine Aufgabe der vorliegenden Erfindung, ein nachhaltigeres Herstellungsverfahren für Bisphenol-A im industriellen Maßstab bereitzustellen, welches die Nutzung regenerativer Rohstoffe ermöglicht. Zudem war es eine weitere Aufgabe der Erfindung, eine Methode zur Herstellung von Bisphenol-A bereitzustellen, mit deren Hilfe trotz möglicher Schwankungen in der Verfügbarkeit regenerativer Energie ein emissionsarmes Bisphenol-A für die Herstellung von Polycarbonaten erreicht werden kann. Unter "regenerativer Energie" versteht der Fachmann Energie aus einer Energiequelle, die sich nicht erschöpft, wie z.B. Windkraft, Wasserkraft, Bioenergie (z.B. Verstromung von Biogas oder Biomasse) oder Sonnenenergie.

Unter "Nachhaltigkeit" eines Verfahrens versteht der Fachmann in Anwendung der durch die UN geprägte Nachhaltigkeits-Definition ("sustainable development") gemäß Brundtlandbericht der "Weltkommission für Umwelt und Entwicklung", dass durch die Ausführung des Verfahrens in der Gegenwart ein möglichst geringer bis gar kein Beitrag geleistet wird, dass künftige Generationen der Menschheit ihre eigenen Bedürfnisse nicht mehr befriedigen können, insbesondere Bedürfnisse mit Blick auf die Nutzung von Ressourcen wie z.B. fossiler Rohstoffe und insbesondere mit Blick auf die Schonung des Lebensraumes, wie z.B. den Schutz der Erdatmosphäre. Die Erfindung hat somit zur Aufgabe, die Produktion von Bisphenol-A, sowie daraus hergestelltem Polycarbonat nachhaltiger als die aus dem Stand der Technik bekannten Produktionsmethoden zu gestalten. Der Beitrag der Produktion von Bisphenol-A und Polycarbonat zu einer sinkenden Befriedigung der Bedürfnisse zukünftiger Generationen soll vermindert bis vermieden werden.

Ein erster Gegenstand der Erfindung ist daher ein Verfahren zur Herstellung von Bisphenol-A für die Herstellung von Polycarbonat, enthaltend zumindest die Schritte:

a) Bereitstellung von Aceton, welches ein Verfahrensprodukt eines Verfahrens enthaltend mindestens folgende Schritte ist:

i) Bereitstellung von Methanol, welches ein Verfahrensprodukt eines Verfahrens mit mindestens folgenden Schritten ist:
i-1) Umsetzung von CO zu Methanol, wobei das CO ein Verfahrensprodukt mindestens einer partiellen Reduktion von $CO_2$ zu CO und/oder ein Verfahrensprodukt mindestens einer partiellen Oxidation von organischem Material zu CO ist, wobei mindestens ein organisches Material ausgewählt wird aus mindestens einer organischen, festförmigen Verbindung, Methan aus biologischer Quelle oder Mischungen daraus;

ii) Synthese von Aceton aus zuvor bereitgestelltem Methanol durch zumindest folgende Schritte:

Bereitstellung von Propen als Produkt eines Verfahrens mit zumindest folgenden Schritten:

ii-1) Umsetzung des zuvor bereitgestellten Methanols zu einer Produktmischung, enthaltend Dimethylether, Wasser und Methanol;

ii-2) Umwandlung von Ausgangsmaterial, enthaltend Dimethylether, Wasser und Methanol jeweils aus der vorgenannten Produktmischung, bei einer Temperatur von mehr als 200°C durch Kontakt mit einem Katalysator, vorzugsweise mit mindestens einer Zeolith-Verbindung, zu Propen;

Umsetzung des Propens durch zumindest

ii-3) Oxidation des Propens zu Aceton;

b) Bereitstellung von Phenol, welches ein Verfahrensprodukt mindestens eines Herstellverfahrens ist, das zumindest folgende Schritte enthält:

i) Bereitstellung mindestens einer organischen, aromatischen Verbindung gemäß Formel (I),

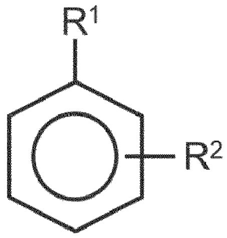

(I)

worin

$R^1$ für ein Wasserstoffatom, eine Hydroxylgruppe, Methyl, Carboxyl, Carboxylat oder $-CHR^3R^4$ steht, worin $R^3$ und $R^4$ unabhängig voneinander für eine $(C_1-C_3)$-Alkylgruppe stehen oder gemeinsam mit dem Restmolekül einen aliphatischen Kohlenwasserstoffzyklus bilden; und

$R^2$ für ein Wasserstoffatom, Methyl, Carboxyl oder Carboxylat steht, mit der Maßgabe, dass falls $R^1$ für $-CHR^3R^4$ steht, $R^2$ ein Wasserstoffatom bedeutet und falls $R^1$ für eine Hydroxylgruppe steht, $R^2$ für ein Wasserstoffatom, Carboxyl oder Carboxylat steht,

wobei die besagte organische, aromatische Verbindung Verfahrensprodukt einer Umwandlung mindestens eines Ausgangsstoffes ausgewählt aus mindestens einer Verbindung aus der Gruppe, die gebildet wird aus polymeren organischen Verbindungen, Kohlehydraten und Methan aus biologischer Quelle, ist;

und falls in besagter organischer, aromatischer Verbindung aus Schritt i) gemäß Formel (I) $R^1$ nicht für eine Hydroxylgruppe steht und $R^2$ nicht für ein Wasserstoffatom steht

ii) mindestens einen der Schritte ii-1) und/oder ii-2) und/oder iii-3)

ii-1) Umsetzung der organischen, aromatischen Verbindung aus Schritt i) mit Propen unter Erhalt einer Propan-2-yl substituierten aromatischen, organischen Verbindung als Produkt;

ii-2) Oxidation der organischen, aromatischen Verbindung aus Schritt i) und/oder des Produkts aus Schritt ii-1), wobei mindestens eine oxidierte, aromatische, organische Verbindung erhalten wird;

ii-3) Abspaltung mindestens eines kohlenstoffhaltigen Substituenten der organischen, aromatischen Verbindung aus Schritt i) und/oder der oxidierten, organischen, aromatischen Verbindung aus der Oxidation gemäß Schritt ii-2);

c) Umsetzung einer Mischung, enthaltend das bereitgestellte Aceton und das bereitgestellte Phenol, zu Bisphenol-A.

Eine "katalysierte Reaktion" oder "katalytische Umsetzung" findet erfindungsgemäß unter Einsatz eines Katalysators statt, der die Produktbildung aus mindestens einem Reaktanden (z.B. Kohlendioxid oder Methanol) im Vergleich zur gleichen Reaktion unter gleichen Reaktionsbedingungen aber in Abwesenheit des Katalysators durch Herabsetzung der aufzuwendenden Energie und/oder durch eine Selektivitätssteigerung unter Erhöhung der Produktausbeute katalysiert. Beispielsweise ist die Umwandlung gemäß Schritt a) ii-2) eine katalytische Umsetzung des besagten Ausgangsmaterials zu Propen.

In Schritt a) des erfindungsgemäßen Verfahrens wird Aceton bereitgestellt. Für die Bereitstellung des Acetons ist es erfindungsgemäß ausreichend, wenn das bereitgestellte Aceton ein tatsächliches Verfahrensprodukt mindestens der unter a) genannten Schritte i) und ii) ist. Dies bedeutet, dass es für die Ausführung des Schrittes der Bereitstellung des Acetons bereits ausreicht, das besagte Aceton lediglich im Rahmen einer Belieferung als Rohstoff aus einem Lagerbehälter oder aus einer Zuleitung zu entnehmen, um dieses zumindest dem Schritt c) des erfindungsgemäßen

Verfahrens zuzuführen. In diesem Falle führt der Bisphenol-A-Produzent als Ausführender des erfindungsgemäßen Verfahrens die unter a) des erfindungsgemäßen Verfahrens genannten Schritte i) und ii) zur Herstellung des Acetons nicht selbst aus, sondern er sorgt nur dafür, dass das bereitgestellte Aceton durch Anwendung mindestens der unter a) genannten Schritte i) und ii) hergestellt wurde und somit deren Verfahrensprodukt ist.

Ebenso ist es erfindungsgemäß möglich, wenn zur Bereitstellung des Acetons die mindestens unter a) genannten Schritte i) und ii) zur Herstellung des Acetons als integrale Schritte eines erfindungsgemäßen Verfahrens zur Herstellung von Bisphenol-A durch den Bisphenol-A-Produzenten ausgeführt werden und dabei erhaltenes Aceton, gegebenenfalls nach einer Zwischenlagerung in einem Lagerbehälter, zumindest dem Schritt c) des erfindungsgemäßen Verfahrens zugeführt wird.

Die Möglichkeiten der Bereitstellung, i.e. Belieferung oder eigene Herstellung des speziellen Acetons durch den Bisphenol-A-Produzenten, gelten jeweils auch für die nachfolgend genannten Ausführungsformen der unter a) genannten Schritte i) und ii).

Der im erfindungsgemäßen Verfahren unter a) genannte Schritt i) erfordert die Bereitstellung von Methanol, welches ein Verfahrensprodukt eines Verfahrens mit mindestens folgenden Schritten ist:

i-1) Umsetzung von CO zu Methanol, wobei das CO ein Verfahrensprodukt mindestens einer partiellen Reduktion von $CO_2$ zu CO und/oder ein Verfahrensprodukt mindestens einer partiellen Oxidation von organischem Material zu CO ist, wobei mindestens ein organisches Material ausgewählt wird aus mindestens einer organischen, festförmigen Verbindung, Methan aus biologischer Quelle oder Mischungen daraus.

Gemäß vorliegender Erfindung ist ein Material oder eine chemische Verbindung organisch, wenn das Material / die chemische Verbindung mindestens eine kovalente Kohlenstoff-Wasserstoff-Bindung enthält.

Als festförmig ist ein Stoff bzw. ein Material definiert, wenn der Stoff bei 25°C und 1013 mbar als Feststoff vorliegt.

Für die Bereitstellung des Methanols ist es gemäß vorliegender Erfindung ausreichend, wenn das Methanol ein tatsächliches Verfahrensprodukt mindestens der unter i-1) genannten Umsetzung ist, wobei für diese Umsetzung Kohlenmonoxid (Kohlenmonoxid auch als CO bezeichnet) genutzt wird, welches wiederum ein Verfahrensprodukt eines speziellen Verfahrens ist. Dies bedeutet, dass es für die Ausführung des Schrittes der Bereitstellung des Methanols bereits ausreicht, das besagte Methanol lediglich im Rahmen einer Belieferung als Rohstoff aus einem Lagerbehälter oder aus einer Zuleitung zu entnehmen. In diesem Falle führt der Bisphenol-A-Produzent als Ausführender des erfindungsgemäßen Verfahrens oder der Lieferant des für das erfindungsgemäße Verfahren bereitgestellte Acetons die vorgenannte Umsetzung zur Herstellung des Methanols nicht selbst aus, sondern er sorgt nur dafür, dass das für die Bereitstellung des Acetons bereitgestellte Methanol durch Anwendung mindestens des besagten Schritts i-1) hergestellt wurde und somit deren Verfahrensprodukt ist.

Ebenso ist es erfindungsgemäß möglich, wenn zur Bereitstellung des Methanols mindestens vorgenannte Umsetzung zur Herstellung des Methanols als integraler Schritt des erfindungsgemäßen Verfahrens zur Herstellung von Bisphenol-A durch den Bisphenol-A-Produzenten oder den Lieferanten des bereitgestellten Acetons ausgeführt werden und das dabei erhaltene Methanol, gegebenenfalls nach einer Zwischenlagerung in einem Lagerbehälter, der unter Schritt a) des Verfahrens vorgesehenen Synthese von Aceton gemäß Schritt ii) unter Ausführung zumindest der Schritte ii-1), ii-2) und ii-3) zugeführt wird.

Die Möglichkeiten der Bereitstellung, i.e. Belieferung oder eigene Herstellung des speziellen Methanols durch den Bisphenol-A-Produzenten, gelten jeweils auch für die nachfolgend genannten Ausführungsformen der Bereitstellung des Methanols.

Für die Herstellung von Methanol werden üblicherweise Kohlenstoffoxide zusammen mit Wasserstoff (das Gemisch auch als Synthesegas bezeichnet) umgesetzt. Dieser Prozess ist exotherm und kann durch folgende Reaktionsgleichungen beschrieben werden:

$$CO + 2H_2 \rightleftharpoons CH_3OH$$

$$CO_2 + 3H_2 \rightleftharpoons CH_3OH + H_2O$$

Beide Reaktionen werden durch die ebenfalls exotherme Water-Gas-Shift Reaktion gekoppelt, welche wie folgt beschrieben werden kann:

$$CO + H_2O \rightleftharpoons CO_2 + H_2$$

Herkömmlicherweise wird für die Methanolsynthese hauptsächlich aus CO und $H_2$ bestehendes Synthesegas eingesetzt, welches wiederum aus Erdgas (fossiles Methan) und Wasserdampf in einem Reformerprozess (steam reforming) hergestellt wird. Die Zusammensetzung des Synthesegases hat starken Einfluss auf die optimale Nutzung. Die Zusammensetzung kann über die Stöchiometriezahl (SZ) beschrieben werden:

$$SZ = \frac{n_{H_2} - n_{CO_2}}{n_{CO} + n_{CO_2}}$$

Für SZ = 2 liegen die Reaktanden im stöchiometrischen Verhältnis gemäß der o.g. Reaktionsgleichungen vor. Real werden jedoch leicht höhere Werte für SZ (2,01-2,1) verwendet, wobei dies durch einen höheren Wasserstoffanteil im Synthesegas realisiert wird (Dittmeyer et al. "Chemische Technik", Band 4, 5. Auflage).

In dem erfindungsgemäßen Verfahren wird das für die Umsetzung zur Methanolbereitstellung benötigte Kohlenmonoxid eben nicht durch die das herkömmliche steam reforming unter Einsatz fossiler Kohlenstoffquellen (Erdgas) bereitgestellt, sondern durch partielle Reduktion von Kohlendioxid und/oder durch partielle Oxidation (und nötigenfalls Gasifizierung) von besagtem organischem Material zu CO.

Bei der partiellen Oxidation von organischem, festförmigem Material (bevorzugt von organischem, polymeren Material) erfolgt gemäß dem erfindungsgemäßen Verfahren die Bereitstellung von CO aus organischem, festförmigen Material (bevorzugt aus gebrauchten Polymer-Abfallfraktionen) und Sauerstoff-haltigem Gas durch Umwandlung des besagten organischen, festförmigen Materials unter partieller Oxidation in ein Gas, enthaltend CO (im Folgenden auch als Gasification bezeichnet).

Durch den Einsatz von Polymer-haltigen Abfallfraktionen, die beispielsweise überwiegend aus PET-, PE-, PP-, Polyurethan- oder Polycarbonat-haltigen Abfallfraktionen bestehen können (nachfolgend Polymer-Abfallfraktion genannt), zur Bereitstellung von CO, werden diese polymer-haltigen Abfallfraktionen einer Wiederverwertung zugefügt und dadurch Bisphenol-A und auch Polycarbonat aus diesem Bisphenol-A mit verbesserter Nachhaltigkeit hergestellt.

Eine "polymere Verbindung" ist ein Molekül mit einer relativen Molmasse (Mw) von mindestens 2000 g/mol, dessen chemische Struktur überwiegend sich mehrfach wiederholende Struktureinheiten umfasst, die sich von einem oder mehreren verschiedenen Molekülen geringerer relativer Molmasse ableiten. Bei den im Rahmen dieser Anmeldung für Polymere bzw. polymere Verbindungen angegebenen mittleren Molmassen handelt es sich - sofern nicht explizit anders gekennzeichnet - stets um gewichtsmittlere Molmassen Mw, die grundsätzlich mittels Gelpermeationschromatographie mit Hilfe eines RI-Detektors bestimmbar sind, wobei die Messung zweckmäßig gegen einen externen Standard erfolgt. Ein "polymeres Material" ist ein Material, enthaltend mindestens eine polymere Verbindung.

Durch die partielle Oxidation werden organisches Material (bevorzugt organisches, festförmiges Material, besonders bevorzugt polymeres, organisches Material, ganz besonders bevorzugt die Polymer-Abfallfraktion) und Sauerstoff zur partiellen Oxidationsreaktion gebracht und zu einem Produktgasgemisch enthaltend Wasserstoff und CO und gegebenenfalls Nebenprodukte umgesetzt. Als Nebenprodukte gelten insbesondere Kohlenwasserstoffe mit 1 bis 8 Kohlenstoffatomen. Im Produktgasgemisch sind u.a. ebenso $CO_2$ und Wasserdampf vorhanden.

Als weiteres Nebenprodukt wird aus dem Gasification-Prozess eine nicht weiter umsetzbare Restfraktion erhalten.

Die für die Gasification benötigte Temperatur wird zumindest teilweise durch partielle Verbrennung (partielle Oxidation) des organischen, festförmigen Materials (bevorzugt des polymeren, organischen Materials, besonders bevorzugt der Polymer-Abfallfraktion) mit einem sauerstoffhaltigen Gas erreicht. Die partielle Oxidation des eingebrachten organischen, festförmigen Materials wird in dem Reaktor bei einer Temperatur von mindestens 400°C durchgeführt. Vorzugsweise wird die partielle Oxidation des Materials in dem Reaktor bei einer Temperatur in einem Temperaturbereich von 600 °C bis 1500°C, insbesondere von 850°C bis 1400°C, weiter bevorzugt von 1100°C bis 1300°C, durchgeführt.

Das als Verfahrensprodukt einer partiellen Oxidation bereitgestellte Kohlenmonoxid kann beispielsweise nach einem Verfahren zur Herstellung von Kohlenmonoxid für die Bereitstellung von Methanol hergestellt worden sein, enthaltend mindestens folgende Schritte

1) Bereitstellung eines sauerstoffhaltigen Gasstroms, enthaltend mindestens 50 Gew.-% Sauerstoffgas,

2) partielle Oxidation von organischem Material (bevorzugt von organischem Material, enthaltend mindestens eine polymere, organische Verbindung), wobei besagtes organisches Material in einen Reaktor einer Vorrichtung eingebracht und dort zumindest durch Zufuhr des besagten sauerstoffhaltigen Gasstromes und von Wärme bei mindestens 400°C unter Bildung eines Produktgases behandelt und das resultierende Produktgas, gegebenenfalls nach mindestens einem weiteren partiellen Oxidationsschritt des Produktgases, als ein Kohlenmonoxid-haltiger Produktgas-Strom gemeinsam mit darin dispergiertem, partikelförmigem Feststoff aus der Vorrichtung ausgebracht wird;

3) der Kohlenmonoxid-haltige Produktgas-Strom einer Reinigung zugeführt wird, in der zumindest

3-1) der Kohlenmonoxid-haltige Produktgas-Strom zur Abtrennung von Feststoff einem Waschschritt zugeführt

wird, worin

(i) Wasser mit dem Kohlenmonoxid-haltigen Produktgas-Strom in Kontakt gebracht wird, wodurch der im Kohlenmonoxid-haltigen Produktgas-Strom dispergierte, partikelförmige Feststoff eine Schlacke bildet und diese Schlacke entfernt und abgeführt wird,
(ii) der von partikelförmigem Feststoff gereinigte, Kohlenmonoxid-haltige Produktgas-Strom ausgebracht wird;

3-2) ein mittels Waschschritt von partikelförmigem Feststoff gereinigter Kohlenmonoxid-haltiger Produktgas-Strom in einem Trockenschritt zumindest einer Abtrennung von Wasser zugeführt, Wasser abgetrennt und der resultierende Kohlenmonoxid-haltige Produktgas-Strom ausgebracht wird,
3-3) ein mittels Abtrennung von Wasser gereinigter Kohlenmonoxid-haltiger Produktgas-Strom zumindest einer Abtrennung von Kohlendioxid zugeführt, Kohlendioxid abgetrennt und der resultierende Kohlenmonoxid-haltige Produktgas-Strom und Kohlendioxid ausgebracht wird;
3-4) ein mittels Abtrennung von Kohlendioxid gereinigter Kohlenmonoxid-haltiger Produktgas-Strom zumindest einer Trenneinheit zur Abtrennung von Kohlenmonoxid zugeführt, eine Abtrennung von Kohlenmonoxid durchgeführt und das resultierende Kohlenmonoxid und ein Wasserstoffgas-haltiges Restgas ausgebracht wird;
3-5) optional ein mittels besagter Trenneinheit abgetrenntes, Wasserstoffgas-haltiges Restgas einer Restgasbehandlung zugeführt, Wasserstoffgas abgetrennt und Wasserstoffgas und ein Endgas ausgebracht wird.

Ein Feststoff ist bekanntermaßen "partikelförmig", wenn er in Form eines körnigen Gemenges aus einer Vielzahl an losen, festförmigen Partikeln des besagten Stoffes vorliegt, das wiederum sogenannte Körner umfasst. Ein Korn ist eine Bezeichnung für die partikulären Bestandteile von Pulvern (Körner sind die losen, festförmigen Partikel), Stäuben (Körner sind die losen festförmigen Partikel), Granulaten (lose, festförmige Partikel sind Agglomerate aus mehreren Körnern) und anderen körnigen Gemengen.

Ein "Reaktor" ist ein Volumen, in dem eine chemischen Umwandlung, z.B. eine partielle Oxidation von polymerer, organischer Verbindung eines Materials, stattfindet. Dies kann für die partielle Oxidation beispielsweise das Volumen eines beheizten Gefäßes sein, in dem sich das Material befindet.

Das für die partielle Oxidation benötigte Sauerstoffgas kann z.B. einer Wasserelektrolyse oder einer Luftzerlegungsanlage entnommen werden. Im Rahmen einer bevorzugten Ausführungsform der partiellen Oxidation, wird zur Bereitstellung des sauerstoffhaltigen Gasstroms eine Elektrolyse von Wasser unter Erhalt von Sauerstoffgas und Wasserstoffgas durchgeführt und das Sauerstoffgas aus dieser Elektrolyse zur Bereitstellung des sauerstoffhaltigen Gasstromes genutzt.

Eine Wasserelektrolyse kann mit Anlagen nach dem Stand der Technik durchgeführt werden. Bekannt und kommerziell erhältlich sind technische Anlagen zur alkalischen Wasserelektrolyse als auch zur Polymerelektrolyt-basierten Elektrolyse, der so genannten PEM-Elektrolyse. Die Prinzipien der Wasserelektrolyse sind beispielhaft in Kapitel 6.3.4 in Volkmar M. Schmidt in "Elektrochemische Verfahrenstechnik" (2003 Wiley-VCH-Verlag; ISBN 3-527-29958-0) beschrieben.

Das in den Reaktor für die partielle Oxidation eingebrachte organische, festförmige Material enthält erfindungsgemäß bevorzugt mindestens eine polymere, organische Verbindung. Die partielle Oxidation sollte in dem Reaktor möglichst gleichmäßig und selektiv stattfinden. Eine Steigerung dieser Parameter kann erzielt werden, wenn im Rahmen einer bevorzugten Ausführungsform jeweils bezogen auf den Zeitpunkt vor der Einbringung das Gewichtsverhältnis des im sauerstoffhaltigen Gasstrom enthaltenen Sauerstoffgases zur polymeren, organischen Verbindung innerhalb eines Gewichtsverhältnisbereiches von 0,4 zu 1,0 bis 1,2 zu 1,0, bevorzugt von 0,6 zu 1,0 bis 0,9 zu 1,0 in den Reaktor eingebracht werden.

Die partielle Oxidation des eingebrachten organischen, festförmigen Materials wird im Rahmen einer bevorzugten Ausführungsform bei einem absoluten Druck von mehr als 1 bar, bevorzugt bei einem absoluten Druck in einem Bereich von 2 bis 80 bar, besonders bevorzugt bei einem absoluten Druck in einem Bereich von 2 bis 50 bar, durchgeführt.

Die partielle Oxidation des organischen, festförmigen Materials kann in mindestens einem der folgenden drei Reaktoren ausgeführt werden: Reaktor für Flugstromvergasung, Reaktor für Wirbelschichtvergasung und Reaktor für Festbettvergasung.

Für die Verwendung in einem Reaktor für Flugstromvergasung muss das organische, festförmige Material, auf eine Korngröße mit einem mittleren Teilchendurchmesser $X_{50,3}$ von < 0,1 mm (Staub) gemahlen werden. Die Zufuhr in den Reaktor erfolgt entweder pneumatisch oder als Aufschlämmung. Die größte Einschränkung dieser Art der partiellen Oxidation für die chemische Wiederverwertung von Abfall ist die Mahlbarkeit und Förderbarkeit von Ausgangsstoffen aus heterogenem Abfall. Eine thermische Behandlung von Biomasse (Torrefaktion) bei 200 - 300 °C unter $O_2$ Ausschluss wird zur Herstellung einer "Biokohle" mit ähnlicher Mahlbarkeit wie Steinkohle verwendet. In einer anderen Variante kann eine vorgeschaltete Pyrolyse zur Herstellung eines pumpbaren Pyrolyseöls verwendet werden. Das durch Abfall-Pyrolyse gewonnene Ö1 kann entweder direkt oder in Form einer mit dem festen Pyrolyserückstand (Pyrolysekoks) vermischten Aufschlämmung partiell oxidiert werden. Diese Prozesskonfiguration wurde von der Firma Noell (Noell-Konversionsverfahren zur Verwertung und Entsorgung von Abfällen, Jürgen Carl EF-Verl. für Energie- und Umwelttechnik, 1994. ISBN: 3924511829) entworfen.

Eine andere Variante stellt die Nutzung eines Reaktors für Wirbelschichtvergasung dar. Die Vergasung von organischem, festförmigen Material, insbesondere von Abfall, in Wirbelschichtreaktoren ist vielfach mit den Technologien EBARA (Showa Denko, Japan), ENERKEM (Enerkem, Edmonton, Kanada) und der Großdemonstration der Hochtemperatur-Winkler-Gasförderung (HTW) durch die Rheinbraun AG (heute RWE) von 1993 bis 1997 in Berrenrath, Deutschland, bekannt. Die Vorbehandlung zur Einspeisung des organischen Materials, bzw. des polymere, organische Verbindungen enthaltenden Materials, in den Reaktor erfordert eine Zerkleinerung auf mit einem mittleren Teilchendurchmesser $X_{50,3}$ von 30 - 80 mm. Die Einspeisung in den Reaktorbehälter erfolgt über Schneckenförderer begrenzt den Vergaserdruck auf maximal 10 bar. Die Technologien von ENERKEM und EBARA ermöglichen die Vergasung von hochkalorischem Abfall (Kunststoffabfall oder Kunststoffreiche Ersatzbrennstoffe. Wirbelschichtvergaser werden bei milden Temperaturen von 700 - 950 °C deutlich unterhalb des Asche-Schmelzpunkts des Einsatzmaterials betrieben, um Anbackungen und Agglomerationen im Reaktor zu vermeiden. Ein weiterer Vorteil dieser milden Reaktortemperatur ist der unvollständige Kohlenstoffumsatz in der Wirbelschicht. Weiterhin enthält das Rohgas aus Reaktoren zur Wirbelschichtvergasern typischerweise erhebliche Mengen an Methan und anderen Kohlenwasserstoffen. Um dies zu kompensieren und eine hohe syn-Gasausbeute von $H_2$ und CO zu gewährleisten, nutzen die Prozesse von ENERKEM und EBARA eine zweite Hochtemperatur-Stufe zur partiellen Oxidation (ca. 1400 °C), die direkt hinter dem Wirbelbett angeordnet ist, um Flugasche zu schmelzen und Kohlenwasserstoffe im Produktgas der ersten Stufe zum finalen Kohlenmonoxid-haltigen Produktgasstrom umzuwandeln. ENERKEM nennt diese zweite Stufe "Thermoreformer", EBARA bezeichnet einen "Hochtemperatur-Vergasungsofen". Die hohe Temperatur dieser zweiten partiellen Oxidationsstufe erhöht die $CO_2$-Produktion.

Das CO für die Bereitstellung des Methanols kann ebenso bevorzugt als Verfahrensprodukt aus der Umwandlung von Methan aus biologischer Quelle mittels partieller Oxidation in einem klassischen Reformerprozess stammen. Im Rahmen einer bevorzugten Ausführungsform einer solchen Bereitstellung ist das CO ein Verfahrensprodukt einer Umsetzung von zumindest Methan aus biologischer Quelle und Wasserdampf (besonders bevorzugt unter Zusatz von $CO_2$), unter Zuführung von Wärmeenergie bei einer Temperatur von mindestens 500°C zu Kohlenmonoxid. Ein darüber hinaus geeigneter, nachhaltiger Reformerprozess mit Zusatz von $CO_2$ wird in der PCT-Patentanmeldung mit der Anmeldenummer PCT/EP2022/052267 beschrieben, auf die ausdrücklich und vollinhaltlich Bezug genommen wird. Dieses Verfahren betrifft die Herstellung von Kohlenmonoxid aus Methan aus biologischer Quelle, Wasserdampf und $CO_2$, enthaltend zumindest die Schritte

Synthese von Kohlenmonoxid in einem Reformerprozess, worin Methan aus biologischer Quelle und Wasserdampf unter Zusatz von mindestens $CO_2$ unter Zuführung von Wärmeenergie bei einer Temperatur von mindestens 500°C zu Kohlenmonoxid-haltigem Produktgas umgesetzt wird,

Reinigung des aus vorgenannter Synthese erhaltenen Kohlenmonoxid-haltigen Produktgases, mindestens durch Abtrennung von $CO_2$ und gegebenenfalls zusätzlich durch mindestens eine Abtrennung ausgewählt aus der Abtrennung von Wasser, der Abtrennung von Wasserstoff oder einer Kombination davon, unter Erhalt von Kohlenmonoxid;

Bereitstellung von $CO_2$ für den besagten Zusatz zum vorgenannten Reformerprozess zumindest aus besagter Abtrennung von $CO_2$ des vorgenannten Reinigungsschritts.

Dabei ist es wiederum bevorzugt, wenn die zur Synthese von Kohlenmonoxid dem Reformerprozess zugeführte Wärmeenergie durch mindestens eine Methode bereitgestellt wird, ausgewählt aus (i) der Verbrennung von Brennstoff, der mittels regenerativer Energie erzeugten Wasserstoff enthält, (ii) der Verbrennung von Brennstoff, der Methan aus biologischer Quelle enthält, (iii) Umwandlung von aus regenerativer Energie erzeugter, elektrischer Energie in Wärme.

Unter "Methan aus biologischer Quelle" (auch als Bio-Methan bezeichnet), versteht der Fachmann in Abgrenzung zu fossilem Methan solches Methan, welches technisch durch Methan-Gärung aus Biomasse gewonnen wird. Die Methan-Gärung ist bekanntermaßen der anaerobe Abbau organischer Stoffe durch Mikroorganismen. Methan aus biologischer Quelle wird beispielsweise in Biogasanlagen hergestellt, worin sowohl organische Abfälle als auch nachwachsende Rohstoffe entsprechend vergoren werden.

Unter "regenerativer Energie" versteht der Fachmann Energie aus einer Energiequelle, die sich nicht erschöpft, wie z.B. Windenergie, Wasserenergie, Bioenergie (z.B. Verstromung von Biogas oder Biomasse) oder Sonnenenergie. Als regenerative Energie eignen sich daher ganz besonders bevorzugt wahlweise Windkraft, Sonnenenergie, Wasserkraft oder Mischungen daraus.

Erfindungsgemäß bevorzugt ist das gemäß i) des erfindungsgemäßen Verfahrensschritts a) bereitgestellte Methanol ein Verfahrensprodukt einer Umsetzung von CO, wobei dieses bei der Umsetzung eingesetzte CO ein Verfahrensprodukt mindestens einer partiellen Reduktion von $CO_2$ zu CO ist, insbesondere ausgewählt aus einer reversewatergas-shift Reaktion unter Einsatz von mittels Elektrolyse (bevorzugt mittels Wasserelektrolyse, i.e. Elektrolyse von Wasser) bereitgestelltem $H_2$, aus einer elektrochemischen Reduktion von $CO_2$ zu CO oder aus Mischungen daraus.

Im Rahmen einer besonders bevorzugten Ausführungsform des erfindungsgemäßen Verfahrens wird das bereitgestellte Methanol als Verfahrensprodukt einer Umsetzung von CO bereitgestellt, wobei das bei der Umsetzung eingesetzte CO ein Verfahrensprodukt mindestens einer partiellen Reduktion eines Gasstroms hergestellt wurde, der mindestens $CO_x$ mit $x = 1$ oder 2 und gegebenenfalls Wasserstoffgas enthält.

Es hat sich weiter als ganz bevorzugt herausgestellt, wenn in einer Ausführungsform des Verfahrens Methanol durch die Umsetzung von $CO_2$ mit oder ohne Wasserstoff gewonnen und für die Bereitstellung von Methanol genutzt wird. Dabei ist es wiederum besonders bevorzugt, wenn die Umsetzung eine elektrochemische Reaktion, eine homogen katalysierte Reaktion oder eine heterogen katalysierte Reaktion ist. Das dafür eingesetzte $CO_2$ stammt in einer weiteren Ausführungsform zusätzlich aus einer weiteren $CO_2$-Quelle, beispielsweise das bei der Bereitstellung von Wärmeenergie emittierte $CO_2$ oder das $CO_2$ aus einer externen $CO_2$-Quelle, z.B. einem Industrieabgas.

Eine "elektrochemische Reaktion" findet erfindungsgemäß durch Anlegen von elektrischem Strom im Reaktionsmedium (z.B. über mindestens eine in das Reaktionsmedium eintauchende Elektrode) in Gegenwart mindestens eines Reaktanden (z.B. Kohlendioxid) statt.

Eine "katalysierte Reaktion" oder "katalytische Umsetzung" wird wie zuvor definiert (*vide supra*). Eine "homogen katalysierte Reaktion" findet erfindungsgemäß unter Einsatz eines homogenen Katalysators und eine "heterogen katalysierte Reaktion" unter Einsatz eines heterogenen Katalysators statt.

Als eine bevorzugte $CO_2$-Quelle dient mindestens eine "externe $CO_2$-Quelle", die $CO_2$ beiträgt, welches nicht durch das erfindungsgemäße Verfahren emittiert wird. Eine externe $CO_2$ Quelle wäre z.B. das $CO_2$, das bei einer Zementherstellung, bei einer $H_2$-Herstellung für die Ammoniaksynthese anfällt, bei einer Fermentation anfällt, bei Verbrennung von Brennstoffen (z.B. einer Abfallverbrennung) im Abgas entsteht, oder $CO_2$, das aus der Luft gewonnenen wird.

Im Rahmen einer besonders bevorzugten Ausführungsform des erfindungsgemäßen Verfahrens wird das Kohlenmonoxid für die Methanolsynthese aus $CO_2$ in einer reverse water-gas shift (RWGS) Reaktionszone hergestellt, durch mindestens eine Umsetzung von Wasserstoff und $CO_2$ zu Kohlenmonoxid. Ein solche Ausführungsform der Kohlenmonoxid-Herstellung wird beispielsweise in dem Dokument WO 2021/089737 A beschrieben, auf das hierin ausdrücklich und vollinhaltlich Bezug genommen wird.

Eine "Reaktionszone" ist der Teil eines Reaktionsraumes, in dem eine chemische Reaktion, z.B. die reverse watergas shift Reaktion, abläuft. Ein "Reaktionsraum" ist ein Volumen, in dem die an einer chemischen Reaktion beteiligten Reaktionspartner zusammengebracht werden und in dem die chemische Reaktion stattfindet. Dies kann für eine chemische Reaktion beispielsweise das Volumen eines Gefäßes sein, in dem sich ein Edukt, z.B. im Falle einer RWGS-Reaktion Kohlendioxid, und dessen Reaktionspartner, im Falle einer RWGS-Reaktion Wasserstoff, gemeinsam befinden und in der Reaktionszone zur Reaktion gebracht werden. Dieses Volumen kann sich beispielsweise in einem Reaktor befinden.

Das für die RWGS-Reaktion genutzte Wasserstoffgas wird bevorzugt mittels Elektrolyse, insbesondere mittels Chloralkalielektrolyse oder Wasserselektrolyse, besonders bevorzugt mittels Wasserelektrolyse, bereitgestellt. Dabei wird wiederum jeweils bevorzugt aus regenerativer Energie (insbesondere aus Wasserkraft, Sonnenenergie oder Windkraft) hergestellte elektrische Energie verwendet.

In dem besonders bevorzugten Verfahren ist das für die Bereitstellung des Methanols bereitgestellte Kohlenmonoxid ein Produkt eines Verfahrens mit mindestens folgenden Schritten:

Bereitstellung eines $CO_2$-Gasstroms,

Reinigung des $CO_2$ Gasstroms von Nebenbestandteilen, insbesondere von Stickoxiden, Schwefelverbindungen, Staub, Wasser, Sauerstoff und HCl wahlweise mittels Adsorption, Gaswäsche oder katalytischer Behandlung unter Erhalt eines gereinigten Kohlendioxids,

Einspeisung von Wasserstoffgas zusammen mit dem gereinigten $CO_2$ Gasstrom in eine RWGS Reaktionszone und Umsetzung der Edukte nach dem Prinzip der RWGS Reaktion zu einem Produktgasgemisch aus Wasserdampf, CO und gegebenenfalls Nebenprodukten, insbesondere niedere Kohlenwasserstoffe, insbesondere bevorzugt Methan,

Abtrennung von nicht umgesetztem Kohlendioxid aus dem aus der Abtrennung erhaltenen Gasgemischs der RWGS Reaktion, insbesondere mittels Aminwäsche, und Rückführung des nicht umgesetzten Kohlendioxids in die RWGS

Reaktion,

Abtrennung des in der RWGS Reaktion nicht umgesetzten Wasserstoffs aus dem nach der Abtrennung erhaltenen Gasgemisch von Kohlenmonoxid und Wasserstoff, insbesondere unter Verwendung einer Coldbox, und wahlweise Rückführung des Wasserstoffs in die RWGS Reaktion,

Ausbringung des verbliebenen Kohlenmonoxids aus der Abtrennung.

Als $CO_2$-Quelle zur Bereitstellung des $CO_2$-Gasstroms dient beispielsweise das bei der Bereitstellung von Wärmeenergie für das erfindungsgemäße Verfahren (z.B. für die RWGS-Reaktion) emittierte $CO_2$, das bei einer Luftzerlegung erhaltene $CO_2$ oder $CO_2$ aus einer weiteren externen $CO_2$-Quelle.

Eine "externe $CO_2$-Quelle" trägt $CO_2$ bei, welches nicht durch das erfindungsgemäße Verfahren oder dessen Ausführungsformen emittiert wird. Eine externe $CO_2$ Quelle wäre z.B. das $CO_2$, das bei einer Zementherstellung, bei Verbrennung von Brennstoffen (z.B. einer Abfallverbrennung) im Abgas entsteht, oder $CO_2$, das aus der Luft gewonnenen wird. Dieses $CO_2$ aus einer externen $CO_2$-Quelle wird im Rahmen einer bevorzugten Ausführungsform des erfindungsgemäßen Verfahrens durch Absorption eines $CO_2$ -Anteils aus (i) Prozessgasen oder Abgasen, die aus mindestens einem Prozess ausgewählt aus Zementherstellung, $H_2$-Herstellung, Verbrennung ausgewählt wird und/oder (ii) aus Luft durch einleiten in Alkalilauge, zum Beispiel Kalilauge, erfolgen. Hierbei bildet sich Kaliumhydrogencarbonat, welches anschließend wieder zu $CO_2$ und Kalilauge thermisch zersetzt werden kann. Das dabei freigesetzte $CO_2$ wird dann dem erfindungsgemäßen Prozess zur Synthese von Kohlenmonoxid zugeführt.

Die RWGS Reaktion wird in der Reaktionszone bevorzugt bei einer Temperatur ≥ 650°C, besonders bevorzugt ≥ 700°C, ganz besonders bevorzugt ≥ 750°C durchgeführt.

Die RWGS Reaktion wird bevorzugt in Gegenwart mindestens eines Katalysators durchgeführt. Dieser ist besonders bevorzugt ausgewählt aus mindestens einer Verbindung aus der Gruppe:

(I) Mischmetalloxide der Formel $A_{(1-w-x)}A'_{w}A''_{x}B_{(1-y-z)}B'_{y}B''_{z}O_{3-delta}$
wobei hier gilt:

A, A' und A" sind unabhängig voneinander ausgewählt aus der Gruppe: Mg, Ca, Sr, Ba, Li, Na, K, Rb, Cs, Sn, Sc, Y, La, Ce, Pr, Nd, Pm, Sm, Eu, Gd, Tb, Dy, Ho, Er, Tm. Yb, Tl , Lu, Ni, Co, Pb, Bi und/oder Cd; und

B, B' und B" sind unabhängig voneinander ausgewählt aus der Gruppe: Cr, Mn, Fe, Bi, Cd, Co, Cu, Ni, Sn. Al, Ga, Sc, Ti, V, Nb, Ta, Mo, Pb. Hf, Zr, Tb. W, Gd. Yb, Mg, Li, Na, K, Ce und/oder Zn; und

$$0 \leq w \leq 0.5;\ 0 \leq x < 0.5;\ 0 \leq y \leq 0.5;\ 0 \leq z \leq 0.5 \text{ und } -1 \leq \text{delta} \leq I\ ;$$

(II) Mischmetalloxide der Formel $A_{(1-w-x)}A'_{w}A''_{x}B_{(1-y-z)}B'_{y}B''_{z}O_{3-delta}$ wobei hier gilt:

A, A' und A" sind unabhängig voneinander ausgewählt aus der Gruppe: Mg, Ca, Sr, Ba. Li, Na, K, Rb, Cs, Sn, Sc, Y, La, Ce, Pr, Nd, Sm, Eu, Gd, Tb, Dy, Ho, Er, Tm, Yb, Tl , Lu, Ni, Co, Pb und/oder Cd; und

B ist ausgewählt aus der Gruppe: Cr, Mn, Fe, Bi, Cd, Co, Cu, Ni, Sn, Al, Ga, Sc, Ti, V, Nb, Ta, Mo, Pb, Hf, Zr, Tb, W, Gd, Yb, Mg, Cd, Zn, Re, Ru, Rh, Pd, Os, Ir und/oder Pt; und

B' ist ausgewählt aus der Gruppe: Re, Ru, Rh, Pd, Os, Ir und/oder Pt; und B" ist ausgewählt aus der Gruppe: Cr, Mn, Fe, Bi, Cd, Co, Cu, Ni, Sn, Al, Ga, Sc, Ti, V, Nb, Ta, Mo, Pb, Hf, Zr, Tb. W. Gd, Yb. Mg, Cd und/oder Zn; und

$$0 \leq w \leq 0{,}5;\ 0 \leq x \leq 0{,}5;\ 0 \leq y \leq 0{,}5;\ 0 \leq z \leq 0.5 \text{ und } -I \leq \text{delta} \leq 1\ ;$$

(III) Mischungen von wenigstens zwei verschiedenen Metallen M1 und M2 auf einem Träger, welcher ein mit einem Metall M3 dotiertes Oxid von Al, Ce und/oder Zr umfasst;

wobei hier gilt: M1 und M2 sind unabhängig voneinander ausgewählt aus der Gruppe: Re, Ru, Rh, Ir, Os, Pd und/oder Pt; und

M3 ist ausgewählt aus der Gruppe: Sc, Y, La, Ce, Pr, Nd, Sm, Eu, Gd, Tb, Dy, Ho, Er, Tm, Yb und/oder Lu;

(IV) Mischmetalloxide der Formel $LO_x(M_{(y/z)}Al_{(2-y/z)}O_3)_z$; wobei hier gilt:

L ist ausgewählt aus der Gruppe: Na, K, Rb, Cs, Mg, Ca, Sr, Ba, Sc, Y, Sn, Pb, Pd, Mn, In, Tl, La, Ce, Pr, Nd, Sm, Eu, Gd, Tb, Dy, Ho, Er, Tm. Yb und/oder Lu; und

M ist ausgewählt aus der Gruppe: Ti, Zr, Hf, V, Nb, Ta, Cr, Mo, W, Mn, Re, Fe, Ru, Os, Co, Rh. Ir, Ni, Pd. Pt. Zn, Cu, Ag und/oder Au; und

$$1 < x \leq 2;\ 0 < y \leq 12;\ \text{und}\ 4 \leq z \leq 9;$$

(V) Mischmetalloxide der Formel $LO(Al_2O_3)_z$; wobei hier gilt:
L ist ausgewählt aus der Gruppe: Na, K, Rb. Cs, Mg, Ca, Sr, Ba, Sc, Y, Sn, Pb, Mn, In, Tl, La, Ce, Pr, Nd, Sm, Eu, Gd, Tb, Dy, Ho, Er, Tm, Yb und/oder Lu; und

$$4 \leq z \leq 9;$$

(VI) oxidischer Katalysator, der Ni und Ru umfasst.

(VII) Metall M1 und/oder wenigstens zwei verschiedene Metalle M1 und M2 auf und/oder in einem Träger, wobei der Träger
ein Carbid, Oxycarbid, Carbonitrid, Nitrid, Borid, Silicid, Germanid und/oder Selenid der Metalle A und/oder B ist; wobei hier gilt:

M1 und M2 sind unabhängig voneinander ausgewählt aus der Gruppe: Cr, Mn, Fe, Co, Ni, Re, Ru, Rh, Ir, Os, Pd, Pt, Zn, Cu, La, Ce, Pr, Nd, Sm, Eu, Gd, Tb, Dy, Ho, Er, Tm, Yb, und/oder Lu; und

A und B sind unabhängig voneinander ausgewählt aus der Gruppe: Be, Mg, Ca, Sc, I i, V, Cr, Mn, Fe, Co, Ni, Y, Zr, Nb, Mo, Hf, Ta, W, La, Ce, Pr, Nd, Sm, Eu, Gd, Tb, Dy, Ho, Er, Tm, Yb, und/oder Lu;
und/oder

Reaktionsprodukte von (I), (II), (III), (IV), (V), (VI) und/oder (VII) in Gegenwart von Kohlendioxid, Wasserstoff, Kohlenmonoxid und/oder Wasser bei einer Temperatur von ≥ 700 °C.

Das für die Bereitstellung des Acetons in dem erfindungsgemäßen Verfahren bereitgestellte Methanol wird in einer ebenso bevorzugten Ausführungsform des Verfahrens aus Kohlenmonoxid bereitgestellt, wobei das Kohlenmonoxid ein Produkt einer elektrochemischen partiellen Reduktion von Kohlendioxid zu Kohlenmonoxid ist. Im Rahmen dieser Ausführungsform wird zur CO Bereitstellung ein $CO_2$ Gasstrom in eine Elektrolysevorrichtung eingebracht und an einer Elektrode, bevorzugt an einer Gasdiffusionselektrode, (besonders bevorzugt unter Einsatz von aus regenerativer Energie hergestellter elektrischer Energie) zu Kohlenstoffmonoxid reduziert. Diese elektrochemische partielle Reduktion wird nachfolgend auch mit COz-Elektrolyse bezeichnet.

Die COz-Elektrolyse kann beispielsweise eine Hochtemperaturelektrolyse sein, die bei einer Temperatur von mehr als 600°C ggf. auch unter Zusatz von Wasser zur Herstellung von Synthesegas, betrieben wird. Hochtemperaturelektrolysen sind grundsätzlich bekannt und im Markt verfügbar z.B. von Haldor Topsoe, eCOs®. Bei der Hochtemperaturelektrolyse entsteht an der Anode Sauerstoff.

Wird die $CO_2$-Elektrolyse als Niedertemperaturelektrolyse betrieben, so erfolgt die Elektrolyse bei einer Temperatur unter 150°C.

Bei allen $CO_2$-Elektrolysen wird das $CO_2$ Gas dem Kathodenraum zugeführt.

Im Falle der Niedertemperaturelektrolyse wird $CO_2$ insbesondere an einer Gasdiffusionselektrode zu Kohlenmonoxid und ggf. Wasserstoff umgesetzt. Der Fachmann kennt beispielsweise aus der Druckschrift WO 2021/069470 A Elektroden und eine Methode zur Ausführung einer elektrochemischen Reduktion von $CO_2$. Bevorzugterweise wird die elektrochemischen Reduktion von $CO_2$ nach einem Verfahren der WO 2021/069470 A durchgeführt. Auf diese Druckschrift wird hierin ausdrücklich und vollinhaltlich Bezug genommen. In diesem bevorzugten elektrolytischen Verfahren zu Herstellung von Kohlenmonoxid werden Kohlenmonoxid, ggf. Wasserstoff und Chlor durch elektrochemische Umsetzung von Kohlendioxid und Alkalichloridlösung erhalten. Dieses bevorzugte Elektrolyseverfahren ist dadurch gekennzeichnet, dass das Kohlendioxid an einer Gasdiffusionselektrode als Kathode in einer wässrigen Alkalichlorid-haltigen Lösung als Katholyt elektrochemisch reduziert und gleichzeitig Chlor aus einer wässrigen Alkalichlorid-haltigen Lösung als Anolyt anodisch erzeugt wird, wobei das im Katholyt gebildete Alkalisalz der Kohlensäure, ausgewählt aus

Alkalicarbonat, Alkalihydrogencarbonat oder Mischungen daraus anschließend mit Chlorwasserstoff zu Kohlendioxid und Alkalichlorid umgesetzt wird und das hierbei freigesetzte Kohlendioxid in den Kathodenraum zur Gasdiffusionselektrode und das erzeugte Alkalichlorid wahlweise in den Anodenraum und/oder in den Kathodenraum zurückgeführt werden.

Nach den bekannten Prinzipien kann bei der Niedertemperatur-Elektrolyse auch ein MEA (Membran-Electrode-Assembly) Konzept zum Einsatz kommen. Hierbei wird auf die Membran ein Katalysator aufgebracht. Eine davor gelagerte Gasdiffusionsschicht reguliert den Gas- und Flüssigkeitstransport. Dies kann sowohl auf der Anoden- als auch auf der Kathodenseite erfolgen. Weiterhin besteht die Möglichkeit eine Gasdiffusionselektrode in direktem Kontakt mit der Membran zu bringen.

Die eingesetzte Gasdiffusionselektrode kann in einer zero-gap als auch in einer finite-gap Anordnung in der Elektrolysezelle verbaut sein. Eine bevorzugte Anordnung für eine Niedertemperaturelektrolyse von $CO_2$ wird in der Druckschrift WO 2020/057998 A1 beschrieben, auf die ausdrücklich und vollinhaltlich Bezug genommen wird.

Dem Kathodenraum, bzw. der darin installierten Gasdiffusionselektrode, kann ein Überschuss an $CO_2$ zugeführt werden. Überschuss bedeutet, mehr $CO_2$ einzuleiten als für die stöchiometrische Umsetzung aufgrund des fließenden elektrischen Stromes notwendig wäre. Somit gelangt aus dem Kathodenraum eine Gasmischung bestehend aus nicht umgesetzten $CO_2$, CO und $H_2$.

Im Rahmen einer weiteren Ausführungsform der COz-Elektrolyse ist es bevorzugt, wenn die dafür verwendete elektrische Energie aus regenerativer Energie hergestellte elektrische Energie, insbesondere aus Windkraft, Sonnenenergie oder Wasserkraft hergestellte elektrische Energie, ist.

Sofern das für die Bereitstellung des Methanols genutzte Kohlenmonoxid ein Verfahrensprodukt mindestens eines der vorgenannten Verfahren ist, kann für die Umsetzung des CO zu Methanol mindestens eines der dem Fachmann dafür bekannten Verfahren ausgehend von CO-haltigem Synthesegas genutzt werden.

Sollte aus Schritt ii-1) nicht ohnehin direkt CO im Gemisch mit ausreichend Wasserstoff als Synthesegas entstehen, so wird bei Mangel an einer ausreichenden Menge Wasserstoffgas dem CO vor der Umsetzung zu Methanol Wasserstoffgas unter Erhalt von Synthesegas beigemischt. Dieses im Rahmen einer Bereitstellung von Methanol dem CO zusätzlich beigefügte Wasserstoffgas wird bevorzugt durch Elektrolyse, insbesondere durch Chloralkalielektrolyse oder Wasserelektrolyse, bereitgestellt. Die Wasserelektrolyse kann mit Anlagen nach dem Stand der Technik durchgeführt werden. Bekannt und kommerziell erhältlich sind technische Anlagen zur alkalischen Wasserelektrolyse als auch zur Polymerelektrolyt-basierten Elektrolyse, der so genannten PEM-Elektrolyse. Die Prinzipien der Wasserelektrolyse sind beispielhaft in Kapitel 6.3.4 in Volkmar M. Schmidt in "Elektrochemische Verfahrenstechnik" (2003 Wiley-VCH-Verlag; ISBN 3-527-29958-0) beschrieben. Im Rahmen einer weiteren Ausführungsform ist es bevorzugt, wenn die für die Elektrolyse zur Wasserstoffherstellung verwendete elektrische Energie aus regenerativer Energie hergestellte elektrische Energie, insbesondere aus Windkraft, Sonnenenergie oder Wasserkraft hergestellte elektrische Energie, ist.

Besonders bevorzugt ist eine Ausführungsform, worin das für das erfindungsgemäße Verfahren bereitzustellende Methanol entsprechend als Verfahrensprodukt unter Verwendung regenerativer Energie hergestellt wurde, vorzugsweise an einem geografischen Ort mit guter Verfügbarkeit von regenerativer Energie, um anschließend entweder dieses Methanol durch Lieferung per Transport in Behältern oder per Methanolstrom im Rahmen eines kontinuierlichen Prozesses für das erfindungsgemäße Verfahren bereitzustellen. Für die Bereitstellung im Rahmen eines kontinuierlichen Prozesses steht die Methanolherstellung bevorzugt in Fluidverbindung mit der Herstellung des Acetons, z.B. über eine Rohrleitung.

Anlagen für die Methanolproduktion können am Markt lizenziert werden, beispielsweise bei der Firma Air Liquide, Johnson Matthey und bei weiteren Firmen. Eine Übersicht zur klassischen Methanolproduktion aus Synthesegas ist beispielsweise in Ott et al., Methanol in: Ullmann's Encyclopedia of industrial chemistry, 2012, Wiley-VCH Verlag, Weinheim (doi 10.1002/14356007.a16_465.pub3) publiziert, auf die ausdrücklich und vollinhaltlich Bezug genommen wird.

Das wie zuvor beschrieben gemäß Schritt a) i) bereitgestellte Methanol wird in Schritt a) ii) des erfindungsgemäßen Verfahrens zur Bereitstellung des Acetons durch ein Verfahren, enthaltend zumindest folgende Schritte, zu Aceton umgesetzt:

Bereitstellung von Propen als Produkt eines Verfahren mit zumindest folgenden Schritten:

ii-1) Umsetzung des zuvor bereitgestellten Methanols zu einer Produktmischung, enthaltend Dimethylether, Wasser und Methanol;

ii-2) Umwandlung von Ausgangsmaterial, enthaltend Dimethylether, Wasser und Methanol jeweils aus der vorgenannten Produktmischung, bei einer Temperatur von mehr als 200°C durch Kontakt mit einem Katalysator, vorzugsweise mit mindestens einer Zeolith-Verbindung, zu Propen;

Umsetzung des Propens durch zumindest
ii-3) Oxidation des Propens zu Aceton.

Die Schritte ii-1) und ii-2) sind Teil des Methanol-to-Propylene-Prozesses (auch MTP-Prozess genannt). Der MTP-Prozess wurde zuerst von der Firma Lurgi entwickelt.

In einem MTP-Prozess wird beispielsweise gasförmiges Methanol in einem Reaktor an einem Katalysator zu einer Produktmischung enthaltend Dimethylether, Wasser und Methanol im Sinne des Schritts ii-1) des erfindungsgemäßen Verfahrens umgesetzt. Als Reaktor eignen sich dafür beispielsweise ein Festbett-Reaktor oder ein Wirbelschichtreaktor.

Im Rahmen einer bevorzugten Ausführungsform des erfindungsgemäßen Verfahrens, erfolgt die Umsetzung des bereitgestellten Methanols gemäß Schritt a) ii-1) durch Kontaktieren des Methanols, bevorzugt des Methanols in der Gasphase, mit einem Katalysator.

Als besonders bevorzugter Katalysator zur Umwandlung des Methanols in Schritt ii-1) eignet sich beispielsweise $Al_2O_3$-Granulat, wie beschrieben in den Druckschriften EP 0 448 000 B1 oder DE 197 23 363 A1.

Vor der Umwandlung des Methanols in Schritt a) ii-1) wird das bereitgestellte Methanol bevorzugt auf eine Temperatur in einem Bereich von 200 °C bis 350°C gebracht und die Umwandlung mit diesem entsprechend temperierten Methanol durchgeführt. Die Produktmischung enthaltend Dimethylether, Methanol und Wasser weist nach der Umwandlung wiederum bevorzugt eine Temperatur von 350°C bis 450°C auf.

Die Produktmischung aus Schritt ii-1), insbesondere in Form eines Gasstroms, wird, gegebenenfalls nach der Durchführung von optionalen Reinigungsschritten, als Ausgangsmaterial für Schritt ii-2) beispielsweise in einen weiteren Reaktor eingebracht und dort an einem Katalysator zu Propen umgesetzt. Als Katalysator eignet sich beispielsweise der von der Firma Clariant unter dem Handelsnamen MTPROP® vertriebenen Katalysator, oder eine Zeolith-Verbindung als Katalysator, wie er beispielsweise in der Druckschrift US 7,015,369 B2 in Spalte 1, Zeilen 43-52, sowie in deren Beispielen 1 und 2 beschrieben ist. Vorzugsweise wird im Rahmen einer weiteren Ausführungsform des erfindungsgemäßen Verfahrens das Ausgangsmaterial durch Kontakt an einer Zeolith-Verbindung als Katalysator zu Propen umgewandelt.

Es ist bevorzugt, wenn in Schritt ii-2) das Ausgangsmaterial bei einer Temperatur von 350°C bis 600°C, insbesondere bei einer Temperatur von 380°C bis 550°C, umgesetzt wird.

Das durch Schritt ii-2) des erfindungsgemäßen Verfahrens bereitgestellte Propen wird, gegebenenfalls nach der Abtrennung von Nebenbestandteilen, gemäß Schritt ii-3) zu Aceton oxidiert. Zudem kann es im Rahmen einer Ausführungsform des Schritts b) des erfindungsgemäßen Verfahrens wie weiter unten ausgeführt bevorzugt sein, durch Schritt ii-2) bereitgestelltes Propen in der Bereitstellung von Phenol gemäß Schritt b) über eine Cumol Zwischenstufe in einer Cumol-Oxidation unter Erhalt von Phenol und Aceton zu nutzen (*vide infra*).

Der Oxidationsschritt ii-3) des Propens gemäß erfindungsgemäßem Verfahren kann beispielsweise nach mindestens einer der folgenden Methoden durchgeführt werden:

1) gemäß Wacker-Hoechst-Prozess durch Umsetzung von Propen und Wasser an einem Katalysatorsystem aus Palladium(II)chlorid und Kupfer(II)chlorid zu Aceton;

2) Hydroxylierung von Propen mit Wasser zu 2-Propanol und anschließender katalytischer Dehydrogenierung von 2-Propanol zu Aceton;

3) Umsetzung des Propens zu Cumol und Durchführung des Cumolhydroperoxid-Verfahrens (Hock-Prozess) oder Umsetzung des Propens zu Diisopropylbenzol und Durchführung des Cumolhydroperoxidverfahrens mit Diisopropylbenzolhydroperoxid anstelle von Cumolhydroperoxid, jeweils unter Erhalt von Aceton.

Diese Methoden und deren Durchführung sind dem Fachmann geläufig, wie beispielsweise in der Publikation Weber et al., Acetone in: Ullmann's Encyclopedia of industrial chemistry, 2013, Wiley-VCH Verlag, Weinheim (doi 10.1002/14356007.a01_079.pub4) beschrieben, worauf ausdrücklich und vollinhaltlich Bezug genommen wird.

Zudem erfolgt im Rahmen des erfindungsgemäßen Verfahrens neben der Bereitstellung des besagten Acetons die Bereitstellung von Phenol in einem Schritt b), wobei das bereitgestellte Phenol ein Verfahrensprodukt mindestens eines Herstellverfahrens ist, das zumindest folgende Schritte enthält:

i) Bereitstellung mindestens einer organischen, aromatischen Verbindung gemäß Formel (I),

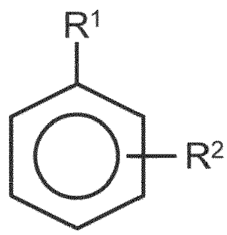

(I)

worin

$R^1$ für ein Wasserstoffatom, eine Hydroxylgruppe, Methyl, Carboxyl, Carboxylat oder - $CHR^3R^4$ steht, worin $R^3$ und $R^4$ unabhängig voneinander für eine ($C_1$-$C_3$)-Alkylgruppe stehen oder gemeinsam mit dem Restmolekül einen aliphatischen Kohlenwasserstoffzyklus bilden; und

$R^2$ für ein Wasserstoffatom, Methyl, Carboxyl oder Carboxylat steht, mit der Maßgabe, dass falls $R^1$ für -$CHR^3R^4$ steht, $R^2$ ein Wasserstoffatom bedeutet und falls $R^1$ für eine Hydroxylgruppe steht, $R^2$ für ein Wasserstoffatom, Carboxyl oder Carboxylat steht,

wobei die besagte organische, aromatische Verbindung Verfahrensprodukt einer Umwandlung mindestens eines Ausgangsstoffes ausgewählt aus mindestens einer Verbindung aus der Gruppe, die gebildet wird aus polymeren organischen Verbindungen, Kohlehydraten und Methan aus biologischer Quelle, ist;

und falls in besagter organischer, aromatischer Verbindung aus Schritt i) gemäß Formel (I) $R^1$ nicht für eine Hydroxylgruppe steht und $R^2$ nicht für ein Wasserstoffatom steht

ii) mindestens einen der Schritte ii-1) und/oder ii-2) und/oder iii-3)

ii-1) Umsetzung der organischen, aromatischen Verbindung aus Schritt i) mit Propen unter Erhalt einer Propan-2-yl substituierten aromatischen, organischen Verbindung als Produkt;

ii-2) Oxidation der organischen, aromatischen Verbindung aus Schritt i) und/oder des Produkts aus Schritt ii-1), wobei mindestens eine oxidierte, aromatische, organische Verbindung erhalten wird;

ii-3) Abspaltung mindestens eines kohlenstoffhaltigen Substituenten der organischen, aromatischen Verbindung aus Schritt i) und/oder der oxidierten, organischen, aromatischen Verbindung aus der Oxidation gemäß Schritt ii-2).

Unter "Phenol" wird erfindungsgemäß die Verbindung Monohydroxybenzol verstanden.

In Schritt b) des erfindungsgemäßen Verfahrens wird Phenol bereitgestellt. Für die Bereitstellung des Phenols ist es erfindungsgemäß ausreichend, wenn das bereitgestellte Phenol ein tatsächliches Verfahrensprodukt mindestens der unter b) genannten Schritte i) und ii) ist. Dies bedeutet, dass es für die Ausführung des Schrittes der Bereitstellung des Phenols bereits ausreicht, das besagte Phenol lediglich im Rahmen einer Belieferung als Rohstoff aus einem Lagerbehälter oder aus einer Zuleitung zu entnehmen, um dieses zumindest dem Schritt c) des erfindungsgemäßen Verfahrens zuzuführen. In diesem Falle führt der Bisphenol-A-Produzent als Ausführender des erfindungsgemäßen Verfahrens die unter b) des erfindungsgemäßen Verfahrens genannten Schritte i) und ii) zur Herstellung des Phenols nicht selbst aus, sondern er sorgt nur dafür, dass das bereitgestellte Phenol durch Anwendung mindestens der unter b) genannten Schritte i) und ii) hergestellt wurde und somit deren Verfahrensprodukt ist.

Ebenso ist es erfindungsgemäß möglich, wenn zur Bereitstellung des Phenols die mindestens unter b) genannten Schritte i) und ii) zur Herstellung des Phenols als integrale Schritte eines erfindungsgemäßen Verfahrens zur Herstellung von Bisphenol-A durch den Bisphenol-A-Produzenten ausgeführt werden und dabei erhaltenes Phenol, gegebenenfalls nach einer Zwischenlagerung in einem Lagerbehälter, zumindest dem Schritt c) des erfindungsgemäßen Verfahrens zugeführt wird.

Die Möglichkeiten der Bereitstellung, i.e. Belieferung oder eigene Herstellung des speziellen Phenols durch den Bisphenol-A-Produzenten, gelten jeweils auch für die nachfolgend genannten Ausführungsformen der unter b) genannten Schritte i) und ii).

Die besagte organische, aromatische Verbindung Verfahrensprodukt der Formel (I) gemäß Schritt b) i) wird durch eine Umwandlung mindestens eines Ausgangsstoffes ausgewählt aus mindestens einer Verbindung aus der

Gruppe, die gebildet wird aus polymeren organischen Verbindungen, Kohlehydraten und Methan aus biologischer Quelle, bereitgestellt. Als Ausgangsstoff eignet sich bevorzugt mindestens eine polymere, organische Verbindung, die ausgewählt wird aus mindestens einer homopolymeren oder copolymeren Verbindung aus der Gruppe Lignin, Polyester, Polyamid, Polyurethan (PUR), Polyharnstoff, Polyisocyanurat (PIR), Polycarbonat, bevorzugt aus mindestens einer homopolymeren oder copolymeren Verbindung der Gruppe Polyurethan (PUR), Polyisocyanurat (PIR). Als Ausgangsstoff eignet sich bevorzugt mindestens ein Kohlehydrat, ausgewählt aus Polysaccharid (wie besonders bevorzugt Stärke oder Cellulose), Trisacchid (z.B. Kestose), sowie C-6 Monosaccharid (wie besonders bevorzugt Glucose, Setose oder Mannose), C-5 Monosaccharid (wie besonders bevorzugt Xylose oder Arabinose).

In einer Ausführungsform des Schritts b) wird in Schritt b) i) mindestens eine Verbindung der Formel (I) bereitgestellt, in der $R^1$ und $R^2$ unabhängig voneinander für ein Wasserstoffatom oder eine Methylgruppe stehen, für diese Ausführungsform bevorzugt Toluol und/oder Benzol. Diese mindestens eine Verbindung (insbesondere Benzol und/oder Toluol) wird in einer bevorzugten Ausführungsform durch Pyrolyse des Ausgangsstoffes bei einer Temperatur von 400 bis 1000°C erhalten. Unter einer Pyrolyse versteht der Fachmann eine thermische Zersetzung unter Sauerstoff-armen Bedingungen, wobei die Abwesenheit von Sauerstoff angestrebt wird, damit ein Verbrennung des organischen Materials weitestgehend vermieden wird. Dabei ist es bevorzugt, wenn bei Ausführung der Pyrolyse die Gasphase weniger als 2 Vol.-% Sauerstoff, insbesondere weniger als 1 Vol.-% Sauerstoff, enthält. Entsprechend geeignete Pyrolyseverfahren zur Gewinnung von Xylol, Toluol, sowie von Benzol sind dem Fachmann beispielsweise aus den Druckschriften WO 2020/204707 A1 und US 9,328,297 B1 bekannt.

Im Rahmen einer besonders bevorzugten Ausführungsform der Pyrolyse wird diese mindestens eine Verbindung als ein Verfahrensprodukt eines Verfahrens bereitgestellt, umfassend mindestens folgende Schritte:

Pyrolyse von organischem Material bei einer Temperatur von 600 bis 1000°C unter Erhalt von gasförmigem Pyrolysat und Pyrolyserückstand;

Kontaktieren des gasförmigen Pyrolysates mit einem heterogen Katalysator unter Erhalt von Konversionsprodukt, enthaltend mindestens eine aromatische, organische Verbindung mit einem Molekulargewicht von weniger als 300 g/mol;

Aufarbeitung des Konversionsproduktes unter Erhalt der besagten Verbindung der Formel (I), insbesondere Toluol oder Benzol.

Ein entsprechendes Pyrolyseverfahren zur Bereitstellung wird beispielsweise in der Druckschrift WO 2020/204707 A1 beschrieben, auf die in dieser Anmeldung ausdrücklich und vollinhaltlich Bezug genommen wird. Hierin sind ebenso für den Prozess geeignete heterogene Katalysatoren zur Herstellung des Konversionsproduktes beschrieben. Als organisches Material zur Pyrolyse eignen sich beispielsweise Polysaccharide, Lignin, Holz, Kunststoffabfall oder mindestens eine homopolymere oder copolymere Verbindung aus der Gruppe Polyester, Polyamid, Polyurethan (PUR), Polyharnstoff, Polyisocyanurat (PIR), Polycarbonat.

Im Rahmen einer weiteren Ausführungsform des Schritts b) wird in Schritt b) i) mindestens eine Verbindung der obigen Formel (I) bereitgestellt, in der $R^1$ und $R^2$ unabhängig voneinander für ein Wasserstoffatom oder eine Methylgruppe stehen, besonders bevorzugt Toluol und/oder Benzol, wobei als Ausgangsmaterial Methan aus biologischer Quelle eingesetzt wird. Die Konversion von Methan zu aromatischen Kohlenwasserstoffen wie Benzol sind dem Fachmann geläufig. Aromatischer Kohlenwasserstoff, insbesondere Benzol, wird beispielsweise in einem Wirbelschichtreaktor oder einem Festbettreaktor bei Kontakt von Methan mit einem Katalysator unter Zuführung von Wärme bereitgestellt, wobei der Katalysator durch Imprägnieren von ZSM-5 (einem offenporigen Zeolithen) als Träger mit 4-Gew.-% Molybdän-Verbindung erhalten wurde (B. Cook et al., Applied Catalysis A: General 365 (2009), 34-41). Ebenso wurde in der Druckschrift WO2006/068814 ein optimiertes Verfahren beschrieben, worin die für die Reaktion notwendige Temperatur dem Prozessstrom in zwischengeschalteten Heizabschnitten zugeführt wird. Das Methan aus biologischer Quelle lässt sich im Rahmen dieser Ausführungsform in den Prozessen des Standes der Technik analog zur Bereitstellung von aromatischen Kohlenwasserstoffen, insbesondere von Benzol, nutzen.

Die im Rahmen der Ausführungsformen des Schritts b) bereitgestellte Verbindung der obigen Formel (I), in der $R^1$ und $R^2$ unabhängig voneinander für ein Wasserstoffatom oder eine Methylgruppe stehen, dient beispielsweise als Ausgangsstoff zur Bereitstellung von oxidierter, aromatischer, organischer Verbindung als Verfahrensprodukt von Schritt b) ii-2).

Sofern mindestens eine der Gruppen $R^1$ oder $R^2$ für eine Methylgruppe stehen, werden die vorhandenen Methylgruppen der Verbindung der Formel (I) in Schritt b) ii-2) zu Carboxylgruppen oxidiert. Dies erfolgt beispielsweise bei 100-150°C in Gegenwart von Cobaltnaphthalat und atmosphärischem Sauerstoff. Die dabei erhaltene oxidierte, aromatische, organische Verbindung wird gemäß Schritt b) ii-3) unter Abspaltung von Kohlendioxid decarboxyliert, wodurch Phenol als Verfahrensprodukt erhalten wird. Eine oxidative Decarboxylierung wird beispielsweise in geschmolzenem Edukt (bei z.B. 230-240°C in Gegenwart von atmosphärischem Sauerstoff und Wasserdampf sowie Kupfer(II)benzoat als Katalysator) durchgeführt. Das bei der Decarboxylierung in Schritt b) ii-3) erhaltene Kohlendioxid kann beispielsweise bevorzugt in Schritt a) des erfindungsgemäßen Verfahrens als Kohlendioxidquelle zur Bereitstellung von Methanol genutzt werden.

Sofern beide Gruppen $R^1$ und $R^2$ gemäß obiger Formel (I) für Wasserstoff stehen, kann gemäß Schritt b) ii-2) eine Oxidation unter Einführung einer ringgebundenen Hydroxylgruppe unter Erhalt von Phenol erfolgen. Dies kann beispielsweise mit Sauerstoff, Wasserstoffperoxid oder $N_2O$ erfolgen. Diese Oxidation wird bevorzugt unter Wärmezuführung (z.B. bei 600-800°C) unter Einsatz von Katalysatoren, wie beispielsweise Zeolith (z.B. vom ZSM-5-Typ) oder Titansilicat (TS-1) durchgeführt. Diese direkte Oxidation von Benzol stellt eine mögliche, jedoch keine bevorzugte Ausführungsform des Schritts b) ii-2) dar.

In einer weiteren, besonders bevorzugten Ausführungsform des Schritts b) wird in Schritt b) i) mindestens eine Verbindung der Formel (I) wie zuvor beschrieben bereitgestellt, in der $R^1$ und $R^2$ für Wasserstoff stehen (Benzol), wobei das bereitgestellte Benzol dieser bevorzugen Ausführungsform als organische, aromatische Verbindung in Schritts b) ii-1) Eingang findet, wobei hier die Schritte a) und b) konvergieren, so dass die Schritte b) ii-1) und b) ii-3) Teil des Oxidationsschritts ii-3) aus Schritt a) des Verfahrens sind, in dem das in Schritt a) ii-2) bereitgestellte Propen mit Benzol aus Schritt b) i) zu Cumol umgesetzt wird und anschließend als Oxidation das Cumolhydroperoxid-Verfahren (Hock-Prozess) durchgeführt wird unter Erhalt von Aceton und Phenol als bereitgestelltes Verfahrensprodukt. Daher ist ein Verfahren bevorzugt, indem zur Ausführung des Schritts b) ii-1) das in Schritt b) i) bereitgestellte Benzol in die Oxidation von Propen in Schritt a) ii-3) eingebracht wird und dort mit dem aus Schritt a) ii-2) bereitgestellten Propen zu Cumol umgesetzt wird und anschließend das dabei gebildete Cumol oxidiert wird unter Bereitstellung von Phenol und Aceton.

Die Durchführung des Cumolhydroperoxid-Verfahrens ist dem Fachmann aus diversen Lehrbüchern bekannt und geläufig.

Das gesamte Verfahren gemäß der Ausführungsform mit konvergenten Schritten b) ii-1) und b) ii-3) mit dem Oxidationsschritt ii-3) aus Schritt a) lässt sich somit formulieren als ein Verfahren zur Herstellung von Bisphenol-A für die Herstellung von Polycarbonat, enthaltend zumindest die Schritte:

a) Bereitstellung von Propen, welches ein Verfahrensprodukt eines Verfahrens enthaltend mindestens folgende Schritte ist:

i) Bereitstellung von Methanol, welches ein Verfahrensprodukt eines Verfahrens mit mindestens folgenden Schritten ist:
i-1) Umsetzung von CO zu Methanol, wobei das CO ein Verfahrensprodukt mindestens einer partiellen Reduktion von $CO_2$ zu CO und/oder ein Verfahrensprodukt mindestens einer partiellen Oxidation von organischem Material zu CO ist, wobei mindestens ein organisches Material ausgewählt wird aus mindestens einer organischen, festförmigen Verbindung, Methan aus biologischer Quelle oder Mischungen daraus;

ii) Synthese von Propen aus zuvor bereitgestelltem Methanol durch zumindest folgende Schritte:

ii-1) Umsetzung des zuvor bereitgestellten Methanols zu einer Produktmischung, enthaltend Dimethylether, Wasser und Methanol;

ii-2) Umwandlung von Ausgangsmaterial, enthaltend Dimethylether, Wasser und Methanol jeweils aus der vorgenannten Produktmischung, bei einer Temperatur von mehr als 200°C durch Kontakt mit einem Katalysator, vorzugsweise mit mindestens einer Zeolith-Verbindung als Katalysator, zu Propen;

b) Bereitstellung von Aceton und Phenol, welche ein Verfahrensprodukt mindestens eines Herstellverfahrens ist, das zumindest folgende Schritte enthält:

i) Bereitstellung von Benzol als mindestens eine organische, aromatische Verbindung gemäß Formel (I),

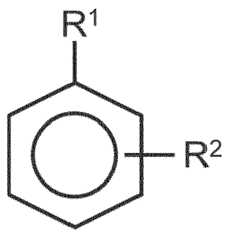

(I)

worin

$R^1$ und $R^2$ für ein Wasserstoffatom stehen,

wobei das Benzol Verfahrensprodukt einer Umwandlung mindestens eines Ausgangsstoffes ausgewählt aus mindestens einer Verbindung aus der Gruppe, die gebildet wird aus polymeren organischen Verbindungen, Kohlehydraten und Methan aus biologischer Quelle, ist;

ii) Ausführung der Schritte ii-1), ii-2) und iii-3)

ii-1) Umsetzung des in Schritt i) bereitgestellten Benzols mit in Schritt a) ii) bereitgestelltem Propen unter Erhalt einer Propan-2-yl substituierten aromatischen, organischen Verbindung, insbesondere Cumol, als Produkt;

ii-2) Oxidation der Propan-2-yl substituierten aromatischen, organischen Verbindung, insbesondere des Cumols, aus Schritt ii-1), wobei mindestens eine aromatische, organische Hydroperoxid-Verbindung, insbesondere Cumolhydroperoxid, erhalten wird;

ii-3) Abspaltung von Aceton als mindestens einen kohlenstoffhaltigen Substituenten der aromatischen, organische Hydroperoxid-Verbindung aus der Oxidation gemäß Schritt ii-2) unter Bereitstellung von Aceton und Phenol;

c) Umsetzung einer Mischung, enthaltend das bereitgestellte Aceton und das bereitgestellte Phenol, zu Bisphenol-A.

Ebenso können ohne Konvergenz der Schritte a) und b) in Ausführung eines Schritts b) ii-3) über eine Oxidation analog des Cumolhydroperoxid-Verfahrens solche in Schritt b) i) bereitgestellten organischen, aromatischen Verbindungen der Formel (I) zu Phenol umgesetzt werden, bei denen $R^1$ für eine Gruppe $CHR^3R^4$ steht, worin $R^3$ und $R^4$ unabhängig voneinander für eine $(C_1-C_3)$-Alkylgruppe stehen oder gemeinsam mit dem Restmolekül einen aliphatischen Kohlenwasserstoffzyklus bilden und unabhängig davon $R^2$ für Wasserstoff stehen. Im Rahmen einer solchen Ausführungsform werden in Schritt b) i) des Verfahrens solche entsprechenden organischen, aromatischen Verbindungen gemäß Formel (I) bereitgestellt, in denen in $R^1$ die Reste $R^3$ und $R^4$ unabhängig voneinander ausgewählt sind aus Methyl, Ethyl, Propyl oder gemeinsam mit dem Restmolekül einen 6-gliedrigen aliphatischen Kohlenwasserstoffring, insbesondere Cyclohexyl, bilden. Verfahren gemäß einem der vorangehenden Ansprüche, dadurch gekennzeichnet, dass gemäß Formel (I) $R^1$ als Rest $-CHR^3R^4$ ausgewählt wird aus Propan-2-yl, sec-Butyl oder Cyclohexyl.

In einer weiteren Ausführungsform des Schritts b) wird in Schritt b) i) mindestens eine Verbindung der Formel (I) bereitgestellt, in der $R^1$ für eine Hydroxylgruppe und $R^2$ für Wasserstoff steht (Phenol). Hierunter fallen insbesondere die erfindungsgemäß bevorzugte Bereitstellung von Phenol als Verfahrensprodukt eines mikrobiologischen Prozesses. Dabei ist wiederum besonders bevorzugt das bereitgestellte Phenol zu erhalten als Produkt eines Verfahrens zur Phenolherstellung in einem rekombinanten Wirt enthaltend die Schritte

Bereitstellung eines rekombinanten Wirtstamms, der durch Verfahren gemäß Druckschrift WO 2014/076113 A1, auf die ausdrücklich und vollinhaltlich Bezug genommen wird, hergestellt wurde,

Inkubation des rekombinanten Wirtstammes unter Fermentationsbedingungen in Gegenwart mindestens eines Saccharids als Ausgangsstoff.

Als Saccharid eignen sich hierfür bevorzugt Polysaccharide wie Disaccharide (z.B. Saccharose), oder Trisaccharide (z.B. Kestose), sowie C-6 Monosaccharide (wie insbesondere Glucose, Setose oder Mannose) und C-5 Monosaccharide (wie Xylose oder Arabinose).

Als Wirtstamm eignen sich beispielsweise solche, ausgewählt aus einem Vertreter der Gruppe die gebildet wird aus Bakterien, Hefen oder Pilzen. Dabei eignen sich wiederum bevorzugt Bakterien des *Escherichia coli* Stammes, wobei *E. coli* BW25113, *E. coli* DH10b, *E. coli* LJ110 besonders bevorzugt geeignet sind. Ebenso bevorzugt eignen sich Bakterien des *Pseudomonas putida* Stammes, insbesondere *Pseudomonas putida* S12.

Die rekombinanten Wirtstämme enthalten die notwendigen, in WO 2014/076113 A1 angegebenen Nukleinsäure Sequenzen.

Durch die Inkubation des Saccharids mit dem rekombinanten Wirt lässt sich beispielsweise Glucose in Phenol umwandeln.

Neben der mikrobiologischen Umwandlung des Ausgangsstoffes kann das Phenol gemäß einer weiteren Ausführungsform des Schritts b) i) aus der Umwandlung der polymeren, organischen Verbindung Lignin bereitgestellt werden. Diese kann durch Depolymerisation des Lignins bei beispielsweise 400°C und bis zu 300 bar Druck in Gegenwart von Stickstoff oder $CO_2$ (vgl. Zurbel et al., Chem. Ing. Tech., 2019, 4, 484-493) und anschließender Umwandlung der erhaltenen Alkylmethoxyphenole, z.B. durch die in Huang et al., ACS Catal. 2018, 8, 11184-11190 beschriebene Methode, erfolgen.

In einer weiteren Ausführungsform des Schritts b) wird in Schritt b) i) mindestens eine Verbindung der Formel (I) bereitgestellt, in der $R^1$ für eine Carboxylgruppe oder Carboxylat steht und $R^2$ für eine Hydroxylgruppe (Salicylsäure/Salicylat) oder für Wasserstoff (Benzoesäure/Benzoat) steht. Daraus wird Phenol gemäß dieser Ausführungsform als Verfahrensprodukt einer Decarboxylierung gemäß Schritt b) ii-3) bereitgestellt. Diese Decarboxylierung kann beispielsweise wie zuvor beschrieben durchgeführt werden (*vide supra*).

Das gemäß Schritt a) bereitgestellte Aceton und das gemäß Schritt b) bereitgestellte Phenol, werden gemischt. Eine Mischung, enthaltend dieses bereitgestellte Aceton und dieses bereitgestellte Phenol, werden im Schritt c) des erfindungsgemäßen Verfahrens zu Bisphenol-A umgesetzt.

Die Umsetzung zu Bisphenol-A erfolgt nach einem dem Fachmann bekannten Verfahren, beispielsweise wie in den Druckschiften WO 00/35847, EP 1 728 777 A1 oder US 2,775,620 beschrieben.

Bevorzugt wird die Umsetzung durch Einsatz mindestens eines Katalysators (insbesondere ausgewählt aus Chlorwasserstoff, Polystyrolsulfonat, vernetztes Polystyrolsulfonat), bei einer Temperatur von 45°C bis 110°C, insbesondere von 50°C bis 80°C, ausgeführt.

Bei der Umsetzung der Mischung ist es wiederum bevorzugt, wenn die eingesetzte Mischung Phenol und Aceton in einem Molverhältnis von Phenol zu Aceton in einem Bereich von 2.1 zu 1 bis 15 zu 1, insbesondere von 5 zu 1 bis 12 zu 1, enthält.

Besonders bevorzugt ist der Katalysator (insbesondere mindestens eine Polystyrolsulfonat-Verbindung) in der Reaktionszone eines Reaktors auf einem Festbett lokalisiert, welches zur Umsetzung mit der besagten Mischung kontaktiert wird.

Ganz besonders bevorzugt erfolgt die Umsetzung von Phenol und Aceton gemäß Schritt c) in Gegenwart mindestens eines sulfonsauren Ionenaustauschers und mindestens eines schwefelhaltigen Cokatalysators zu einem Bisphenol-A enthaltenden Produktgemisch, welches optional nachfolgend unter Erhalt von aufgereinigtem Bisphenol-A aufgearbeitet wird. Als saure Katalysatoren kommen gelförmige (mikroporöse) oder makroporöse, sulfonierte vernetzte Polystyrolharze (saure Ionentauscher) zum Einsatz, die sowohl monodispers als auch heterodispers sein können. Als Vernetzer wird normalerweise Divinylbenzol eingesetzt, aber auch andere Vernetzer wie Divinylbiphenyl können Verwendung finden. Neben dem Katalysator kommt ein Cokatalysator zum Einsatz. Hierbei handelt es sich üblicherweise um ein Thiol, das mindestens eine SH-Funktion trägt und sowohl die Selektivität als auch die Reaktivität der Umsetzung positiv beeinflusst. Der Cokatalysator kann sowohl homogen in der Reaktionslösung gelöst als auch auf dem Katalysator selber fixiert sein. Homogene Cokatalysatoren sind beispielsweise Mercaptopropionsäure, Schwefelwasserstoff, Alkylsulfide oder Alkylsilylthiole, wie beispielsweise Ethylsulfid oder Silylmethanthiol und ähnliche Verbindungen. Fixierte Cokatalysatoren sind Aminoalkylthiole und Pyridylalkylthiole, die ionisch an den Katalysator gebunden sind, wobei die SH-Funktion geschützt sein kann und erst während oder nach Fixierung auf den Katalysator freigesetzt wird, wie beispielsweise bei Dimethylthiazolidin und bei Alkylcarbamoylalkylthioestern. Ebenso kann der Cokatalysator kovalent als Alkyl- oder Arylthiol an den Katalysator gebunden bzw. Bestandteil des Katalysators sein. Es können auch zwei oder mehrere der beschriebenen Cokatalysatoren gemeinsam zum Einsatz kommen.

Die Aufreinigung des nach der Umsetzung erhaltenen Bisphenol-A erfolgt beispielsweise wie in der Druckschrift EP 1 728 777 A1 beschrieben.

In Fig.1 wird das erfindungsgemäße Verfahren exemplarisch illustriert. Darin haben die Bezugszeichen die nachfolgende Bedeutung:

A = organisches Material (als organische, festförmige Verbindung und/oder Methan aus biologischer Quelle) oder $CO_2$
B = Oxidationsmittel (wie beispielsweise Sauerstoffgas) oder Reduktionsmittel (wie beispielsweise Wasserstoffgas oder elektrischer Strom)
C = Produktgasgemisch enthaltend CO
D = Synthesegas
E = Propen
F = Bisphenol-A
G = Ausgangsstoff mindestens aus polymerer, organischer Verbindung oder Kohlehydrat oder Bio-Methan
H = Vorläufer gemäß Formel (I), wenn nicht $R^1$ = Hydroxylgruppe und $R^2$ = Wasserstoffatom
I = Phenol

1 = CO Bereitstellung durch partielle Reduktion von $CO_2$ oder partielle Oxidation des organischen Materials

2 = Umsetzung von CO zu Methanol
3 = Bereitstellung von Methanol aus **2**
4 = Umsetzung des Methanols zu Propen **E** mittels MTP-Prozess
5 = Oxidation des Propens **E** zu Aceton
6 = Bereitstellung von Aceton
7 = Synthese von Bisphenol-A unter Einsatz von bereitgestelltem Aceton und bereitgestelltem Phenol
8 = Bereitstellung von Phenol
9 = Umwandlung des Ausgangsmaterials **G** zu Phenol oder einem Vorläufer davon gemäß Formel (I)
10 = Umwandlung des Vorläufers **H** aus **9** zu Phenol

Pfeile in den Figuren Fig. 1, Fig.2 und Fig.3 symbolisieren den Fluss von Stoffen, Energie oder Wärme zwischen Verfahrensschritten bzw. Vorrichtungsteilen, in denen die entsprechenden Verfahrensschritte ablaufen. Mit gestrichelten Linien gezeichnete Verfahrensmerkmale symbolisieren Verfahrensschritte eines Verfahrens, dessen Verfahrensprodukt die in den Schritten 6 und 8 bereitgestellten Stoffe sind. Ein flächiger Punkt stellt eine Prozessgasaufbereitung incl. optionaler Mischvorrichtung dar.
Fig. 1 zeigt ein Verfahren zur Herstellung von Bisphenol-A **F** für die Herstellung von Polycarbonat, enthaltend zumindest die Schritte:

a) Bereitstellung von Aceton, welches ein Verfahrensprodukt eines Verfahrens enthaltend mindestens folgende Schritte ist:

i) Bereitstellung **3** von Methanol, welches ein Verfahrensprodukt eines Verfahrens mit mindestens folgenden Schritten ist:
i-1) Umsetzung **2** von CO, beispielsweise im Gemisch mit Wasserstoffgas als Synthesegas **D,** zu Methanol, wobei das dafür bereitgestellte CO **3** ein Verfahrensprodukt mindestens einer partiellen Reduktion **1** von $CO_2$ **A** mit Reduktionsmittel **B** zu CO und/oder ein Verfahrensprodukt mindestens einer partiellen Oxidation **1** von organischem Material mit Oxidationsmittel **B** zu CO ist, wobei mindestens ein organisches Material **A** ausgewählt wird aus mindestens einer organischen, festförmigen Verbindung, Methan aus biologischer Quelle oder Mischungen daraus;

ii) Synthese von Aceton aus zuvor bereitgestelltem Methanol **3** durch zumindest folgende Schritte:

Bereitstellung **4** von Propen als Produkt eines Verfahren mit zumindest folgenden Schritten:

ii-1) Umsetzung des zuvor bereitgestellten Methanols **3** zu einer Produktmischung, enthaltend Dimethylether, Wasser und Methanol;

ii-2) Umwandlung von Ausgangsmaterial, enthaltend Dimethylether, Wasser und Methanol jeweils aus der vorgenannten Produktmischung, bei einer Temperatur von mehr als 200°C durch Kontakt mit einem Katalysator, vorzugsweise mit mindestens einer Zeolith-Verbindung als Katalysator, zu Propen **E; und**

Umsetzung von Propen **E** durch zumindest
ii-3) Oxidation **5** des Propens **E** zu Aceton;

b) Bereitstellung **8** von Phenol, welches ein Verfahrensprodukt mindestens eines Herstellverfahrens ist, das zumindest folgende Schritte enthält:

i) Bereitstellung mindestens einer organischen, aromatischen Verbindung gemäß Formel (I),

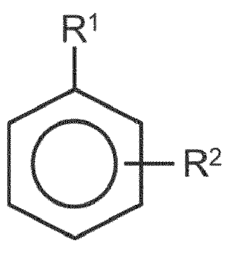

(I)

worin

$R^1$ für ein Wasserstoffatom, eine Hydroxylgruppe, Methyl, Carboxyl, Carboxylat oder $-CHR^3R^4$ steht, worin $R^3$ und $R^4$ unabhängig voneinander für eine $(C_1-C_3)$-Alkylgruppe stehen oder gemeinsam mit dem Restmolekül einen aliphatischen Kohlenwasserstoffzyklus bilden; und

$R^2$ für ein Wasserstoffatom, Methyl, Carboxyl oder Carboxylat steht, mit der Maßgabe, dass falls $R^1$ für $-CHR^3R^4$ steht, $R^2$ ein Wasserstoffatom bedeutet und falls $R^1$ für eine Hydroxylgruppe steht, $R^2$ für ein Wasserstoffatom, Carboxyl oder Carboxylat steht,

wobei die besagte organische, aromatische Verbindung Verfahrensprodukt einer Umwandlung **9** mindestens eines Ausgangsstoffes **G** ausgewählt aus mindestens einer Verbindung aus der Gruppe, die gebildet wird aus polymeren organischen Verbindungen, Kohlehydraten und Methan aus biologischer Quelle, ist;
und falls die besagte organische, aromatischer Verbindung aus Schritt i) gemäß Formel (I) ein Vorläufer **H** ist, in dem $R^1$ nicht für eine Hydroxylgruppe steht und $R^2$ nicht für ein Wasserstoffatom steht

ii) mindestens einen der Schritte ii-1) und/oder ii-2) und/oder iii-3) zur Umwandlung des Vorläufers **10**

ii-1) Umsetzung des Vorläufers **H** aus Schritt i) mit Propen unter Erhalt einer Propan-2-yl substituierten aromatischen, organischen Verbindung als Produkt;

ii-2) Oxidation des Vorläufers **H** aus Schritt i) und/oder des Produkts aus Schritt ii-1), wobei mindestens eine oxidierte, aromatische, organische Verbindung erhalten wird;

ii-3) Abspaltung mindestens eines kohlenstoffhaltigen Substituenten des Vorläufers **H** aus Schritt i) und/oder der oxidierten, organischen, aromatischen Verbindung aus der Oxidation gemäß Schritt ii-2);

c) Umsetzung 7 einer Mischung, enthaltend das bereitgestellte Aceton und das bereitgestellte Phenol, zu Bisphenol-A **F.**

Das aus Schritt c) des Herstellungsverfahrens von Bisphenol-A gemäß erstem Gegenstand der Erfindung bzw. seiner Ausführungsformen erhaltene und gegebenenfalls aufgearbeitete Bisphenol-A eignet sich für die Herstellung von Polycarbonat. Daher ist ein zweiter Gegenstand der Erfindung ein Verfahren zur Herstellung von Polycarbonat, enthaltend zumindest die folgenden Schritte

Herstellung von Bisphenol-A nach dem Verfahren zur Herstellung von Bisphenol-A des ersten Erfindungsgegenstandes;

Herstellung mindestens eines Kohlensäurederivats, ausgewählt aus Phosgen, Kohlensäurediester oder Mischungen daraus;

Umsetzung des besagten Kohlensäurederivats mit mindestens einem Diphenol, mit der Maßgabe, dass zumindest das zuvor hergestellte Bisphenol-A als Diphenol eingesetzt wird.

Die Herstellung des Bisphenol-A wird im ersten Gegenstand der Erfindung beschrieben. Alle Ausführungsformen des ersten Gegenstandes sind gemäß der dort beschriebenen Bevorzugung von einzelnen Verfahrensschritten analog für das Verfahren des zweiten Gegenstandes der Erfindung nutzbar.

Die Herstellung des Kohlensäurederivats Phosgen aus zumindest Kohlenmonoxid und Chlor ist bekannt, beispielsweise aus EP 0 881 986 A1, EP 1 640 341 A2, DE 332 72 74 A1, GB 583 477 A, WO 97/30932 A1, WO 96/16898 A1, oder US 6,713,035 B1. Es hat sich als erfindungsgemäß bevorzugt erwiesen, das Kohlenmonoxid (CO) für die Phosgenherstellung durch ein Verfahren bereitzustellen, in dem das Kohlenmonoxid ein Verfahrensprodukt mindestens einer partiellen Reduktion von $CO_2$ zu CO und/oder ein Verfahrensprodukt mindestens einer partiellen Oxidation von organischem Material zu CO ist, wobei mindestens ein organisches Material ausgewählt wird aus mindestens einer organischen, festförmigen Verbindung, Methan aus biologischer Quelle oder Mischungen daraus. Entsprechend geeignete und bevorzugte Ausführungsformen dieser Bereitstellung von CO sind analog diejenigen, die für Schritt a) i-1) des ersten Gegenstandes der Erfindung beschrieben wurden (*vide supra*).

Die Herstellung des Kohlensäurederivats Kohlensäurediester erfolgt beispielsweise über die Herstellung von Phosgen und die anschließende Umsetzung des Phosgens mit einer organischen Monohydroxyverbindung, wie beispielsweise Phenol, oder über eine katalytische Umsetzung von Phenol und Kohlenmonoxid, sowie Sauerstoff wie sie in der Druckschrift DE 10 2009 011874 A1 beschrieben ist. Der Einsatz von Kohlensäurediphenylester (auch: Diphenylcarbonat) als Kohlensäurediester ist erfindungsgemäß bevorzugt.

Die Herstellung des Polyacarbonats erfolgt in bekannter Weise aus mindestens einem Diphenol, wobei zumindest mittels besagtem Verfahren hergestelltes Bisphenol-A als ein solches Diphenol eingesetzt wird (Diphenol wird nachfolgend auch als Dihydroxyarylverbindung bezeichnet), besagtem Kohlensäurederivat, gegebenenfalls Kettenabbrechern und gegebenenfalls Verzweigern. Zur Herstellung von Polyestercarbonat wird ein Teil des besagten Kohlensäurederivates durch aromatische Dicarbonsäuren oder Derivate der Dicarbonsäuren ersetzt, und zwar je nach Maßgabe der in den aromatischen Polycarbonaten zu ersetzenden Carbonatstruktureinheiten durch aromatische Dicarbonsäureesterstruktureinheiten.

Im Falle der Homopolycarbonate wird in dem erfindungsgemäßen Verfahren des zweiten Gegenstandes nur besagtes Bisphenol-A als Diphenol eingesetzt, im Falle der Herstellung von Copolycarbonaten werden besagtes Bisphenol-A zusammen mit mindestens einem zusätzlichen Diphenol eingesetzt. Bevorzugt nutzbare zusätzliche Diphenole werden ausgewählt aus mindestens einem Diphenol der Gruppe, die gebildet wird aus 4,4'-Dihydroxydiphenyl, 1,1-Bis-(4-Hydroxyphenyl)-phenylethan, 2,2-Bis(3,5-dimethyl-4-hydroxyphenyl)-propan, 1,1-Bis-(4-hydroxyphenyl)-cyclohexan und 1,1-Bis-(4-hydroxyphenyl)-3,3,5-trimethylcyclohexan (Bisphenol TMC (BPTMC)).

Für die Herstellung von Polyestercarbonat geeignete aromatische Dicarbonsäuren sind beispielsweise Orthophthalsäure, Terephthalsäure, Isophthalsäure, tert-Butylisophthalsäure, 3,3'-Diphenyldicarbonsäure, 4,4'-Diphenyldicarbonsäure, 4,4-Benzophenondicarbonsäure, 3,4'-Benzophenondicarbonsäure, 4,4'-Diphenyletherdicarbonsäure, 4,4'-Diphenylsulfondicarbonsäure, 2,2-Bis-(4-carboxyphenyl)-propan, Trimethyl-3-phenylindan-4,5'-dicarbonsäure.

Von den aromatischen Dicarbonsäuren werden besonders bevorzugt die Terephthalsäure und/oder Isophthalsäure eingesetzt.

Derivate der Dicarbonsäuren sind die Dicarbonsäuredihalogenide und die Dicarbonsäuredialkylester, insbesondere die Dicarbonsäuredichloride und die Dicarbonsäuredimethylester.

Der Ersatz der Carbonatgruppen durch die aromatischen Dicarbonsäureestergruppen erfolgt im Wesentlichen stöchiometrisch und auch quantitativ, so dass das molare Verhältnis der Reaktionspartner sich auch im fertigen Polyestercarbonat wiederfindet. Der Einbau der aromatischen Dicarbonsäureestergruppen kann sowohl statistisch als auch blockweise erfolgen. Dem Fachmann sind Verfahren zur Umsetzung von Diphenol und besagtem Kohlensäurederivat bekannt.

Polycarbonat lässt sich beispielsweise in einem Rührreaktor oder Schneckenextruder durch Umesterung von Diphenylcarbonat mit Bisphenoly A in der Schmelze bei ca. 200 - 350°C unter leicht reduziertem Druck (z.B. 0.1 bis 20 kPA) darstellen. Dabei wird Phenol abdestilliert und Polycarbonat in Form von Strängen erhalten, die dann pelletiert oder granuliert werden (V. Serini "Polycarbonates" in: Ullmann's Encyclopedia of Industrial Chemistry, Wiley-VCH, Weinheim, 2000.

Polycarbonat lässt sich beispielsweise ebenso durch Umsetzung von Phosgen mit zumindest Bisphenol-A als mindestens ein Diphenol herstellen. Diese Methode ist erfindungsgemäß bevorzugt. Gemäß einem aus EP 0 304 691 A2 bekannten kontinuierlichen Phasengrenzflächenverfahren zur Herstellung von Polycarbonaten wird eine wässrige Phase aus Diphenol(en) und der gerade notwendigen Menge Alkalihydroxid mit einer Phosgen als besagtes Kohlensäurederivat enthaltenen organischen Phase in einem Rohr unter Verwendung eines statischen Mischers zusammengeführt. Der Phosgenüberschuss ist mit 20 bis 100 Mol-% sehr hoch und die Verweilzeit im Reaktionsrohr für den ersten Reaktionsschritt liegt bei 10 bis 75 s. Mit diesem Verfahren können lediglich Prepolymere mit einem Molekulargewicht von 4000 bis 12000 g/mol hergestellt werden. Anschliessend erfolgt zwingend eine weitere Aufkondensierung unter Verwendung mindestens eines Katalysators, um zum gewünschten Molekulargewicht zu kommen. Geeignete Katalysatoren sind tertiäre Amine und Oniumsalzen. Bevorzugt werden Tributylamin, Triethylamin und N-Ethylpiperidin verwendet.

Der eingesetzte Amin-Katalysator kann offenkettig oder zyklisch sein, besonders bevorzugt sind Triethylamin und N-Ethylpiperidin. Der Katalysator wird vorzugsweise als 1 bis 55 Gew.%-ige Lösung eingesetzt.

Unter Oniumsalzen werden hier Verbindungen wie $NR_4X$ verstanden, wobei R ein Alkyl und/oder Arylrest und/oder ein H sein kann und X ein Anion ist, beispielsweise ein Chlorid-Ion, ein Hydroxid-Ion oder ein Phenolat-Ion.

Das ausreagierte, höchstens noch Spuren an (< 2 ppm) Chlorkohlensäurearylestern enthaltende mindestens zweiphasige Reaktionsgemisch lässt man zur Phasentrennung absitzen. Die wässrige alkalische Phase (Reaktionsabwasser) wird abgetrennt und die organische Phase mit verdünnter Salzsäure und Wasser extrahiert. Die vereinigten Wasserphasen werden der Abwasseraufarbeitung zugeführt, wo Lösungsmittel- und Katalysatoranteile durch Strippen oder Extraktion abgetrennt und rückgeführt werden. Anschließend werden nach der Einstellung eines bestimmten pH-Wertes von z.B. 6 bis 8, z.B. durch Salzsäurezugabe, die gegebenenfalls noch verbleibenden organischen Verunreinigungen wie z.B. Monophenol und/oder nicht umgesetztes Diphenol bzw. nicht umgesetzte Diphenole durch Behandlung mit Aktivkohle entfernt und die Wasserphase der Chloralkalielektrolyse zugeführt.

In einer anderen Variante der Aufarbeitung wird das Reaktionsabwasser nicht mit den Waschphasen vereinigt,

aber nach Strippen oder Extraktion zur Abtrennung von Lösungsmitteln und Katalysatorresten, auf einem bestimmten pH-Wert von z.B. 6 bis 8, z.B. durch Salzsäurezugabe eingestellt und nach Abtrennung der noch verbleibenden organischen Verunreinigungen wie z.B. Monophenol und/oder nicht umgesetztes Diphenol oder nicht umgesetzte Diphenole durch Behandlung mit Aktivkohle der Chloralkalielektrolyse zugeführt.

Die Waschphasen können nach Entfernung der Lösungsmittel- und Katalysatoranteile durch Strippen oder Extraktion gegebenenfalls wieder der Synthese zugeführt werden.

Das Phosgen kann flüssig, gasförmig oder gelöst in einem organischem Lösungsmittel eingesetzt werden.

Nach Eintrag des Phosgens kann es vorteilhaft sein, eine gewisse Zeit die organische Phase und die wässrige Phase zu durchmischen, bevor gegebenenfalls Verzweiger, sofern dieser nicht gemeinsam mit dem Diphenolat dosiert wird, Kettenabbrecher und Katalysator zugegeben werden. Eine derartige Nachreaktionszeit kann nach jeder Dosierung von Vorteil sein. Diese Nachrührzeiten liegen, insofern sie eingelegt werden, zwischen 10 Sekunden und 60 Minuten, bevorzugt zwischen 30 sec. und 40 Minuten, besonders bevorzugt zwischen 1 und 15 min.

Die organische, das Polycarbonat enthaltende Phase muss nun von allen Kontaminationen alkalischer, ionischer oder katalytischer Art gereinigt werden.

Die organische Phase enthält auch nach einem oder mehreren Absetzvorgängen, gegebenenfalls unterstützt durch Durchläufe durch Absetzkessel, Rührkessel, Coalescer oder Separatoren bzw. Kombinationen aus diesen Maßnahmen - wobei gegebenenfalls Wasser in jedem oder einigen Trennschritten unter Umständen unter Verwendung von aktiven oder passiven Mischorganen zudosiert werden kann - noch Anteile der wässrigen alkalischen Phase in feinen Tröpfchen sowie den Katalysator, in der Regel ein tertiäres Amin.

Nach dieser groben Abtrennung der alkalischen, wässrigen Phase wird die organische Phase ein oder mehrmals mit verdünnten Säuren, Mineral-, Carbon- Hydroxycarbon-und/oder Sulfonsäuren gewaschen. Bevorzugt sind wässrige Mineralsäuren insbesondere Salzsäure, phosphorige Säure und Phosphorsäure oder Mischungen dieser Säuren. Die Konzentration dieser Säuren sollte im Bereich 0,001 bis 50 Gew. %, bevorzugt 0,01 bis 5 Gew. % liegen.

Weiterhin wird die organische Phase mit entsalztem oder destilliertem Wasser wiederholt gewaschen. Die Abtrennung der, gegebenenfalls mit Teilen der wässrigen Phase dispergierten, organischen Phase nach den einzelnen Waschschritten geschieht mittels Absetzkessel, Rührkessel, Coalescer oder Separatoren bzw. Kombinationen aus diesen Maßnahmen, wobei das Waschwasser zwischen den Waschschritten gegebenenfalls unter Verwendung von aktiven oder passiven Mischorganen zudosiert werden kann.

Zwischen diesen Waschschritten oder auch nach der Wäsche können gegebenenfalls Säuren, vorzugsweise gelöst im Lösungsmittel welches der Polycarbonatlösung zugrunde liegt, zugegeben werden. Bevorzugt werden hier Chlorwasserstoffgas und Phosphorsäure oder phosphorige Säure verwendet, die gegebenenfalls auch als Mischungen eingesetzt werden können.

Die so erhaltene gereinigte Polycarbonatlösung sollte nach dem letzten Trennvorgang nicht mehr als 5 Gew. %, bevorzugt weniger als 1 Gew. %, ganz besonders bevorzugt weniger als 0,5 Gew. % Wasser enthalten.

Zur Isolierung des Polycarbonats wird in einem ersten Schritt das leichtsiedende Lösungsmittel, beispielsweise Methylenchlorid, gegen ein hochsiedendes Lösungsmittel, beispielsweise Chlorbenzol ausgetauscht. Dies geschieht mittels einer Austauschkolonnne.

Die Isolierung des Polycarbonats aus der Lösung mit dem hochsiedenden Lösungsmittel, beispielsweise Chlorbenzol, kann durch Verdampfen des Lösungsmittels mittels Temperatur, Vakuum oder eines erhitzten Schleppgases erfolgen.

Geschieht die Konzentrierung der Polycarbonatlösung und eventuell auch die Isolierung des Polycarbonats durch Abdestillation des Lösungsmittels, gegebenenfalls durch Überhitzung und Entspannung, so spricht man von einem ‘Flash-Verfahren" siehe auch "Thermische Trennverfahren", VCH Verlagsanstalt 1988, S. 114; wird statt dessen ein geheiztes Trägergas zusammen mit der einzudampfenden Lösung versprüht, so spricht man von einer ‘Sprühverdampfung/Sprühtrocknung" beispielhaft beschrieben in Vauck, "Grundoperationen chemischer Verfahrenstechnik", Deutscher Verlag für Grundstoffindustrie 2000, 11 .Auflage, S. 690. Alle diese Verfahren sind in der Patentliteratur und in Lehrbüchern beschrieben und dem Fachmann geläufig.

Bei der Entfernung des Lösungsmittels durch Temperatur (Abdestillieren) oder dem technisch effektiveren Flash-Verfahren erhält man hochkonzentrierte Polycarbonatschmelzen. Bei dem bekannten Flashverfahren wird eine Polycarbonatlösung wiederholt unter leichtem Überdruck auf Temperaturen oberhalb des Siedepunktes unter Normaldruck erhitzt und diese, bezüglich des Normaldruckes, überhitzten Lösungen anschliessend in ein Gefäß mit niedrigerem Druck, z.B. Normaldruck, entspannt. Es kann dabei von Vorteil sein, die Aufkonzentrationsstufen, oder anders ausgedrückt die Temperaturstufen der Überhitzung nicht zu groß werden zu lassen sondern lieber ein zwei- bis vierstufiges Verfahren zu wählen.

Aus der so erhaltenen hochkonzentrierten Polycarbonatschmelze können die Reste des Lösungsmittels entweder direkt aus der Schmelze mit Ausdampfextrudern (BE-A 866 991, EP-A 0 411 510, US-A 4 980 105, DE-A 33 32 065), Dünnschichtverdampfern (EP-A-0 267 025), Fallfilmverdampfern, Strangverdampfern oder durch Friktionskompaktierung (EP-A-0 460 450), gegebenenfalls auch unter Zusatz eines Schleppmittels, wie Stickstoff oder Kohlendioxid

oder unter Verwendung von Vakuum (EP-A 0 039 96, EP-A 0 256 003, US-A 4 423 207) , entfernt werden, alternativ auch durch anschließende Kristallisation (DE-A 34 29 960) und Ausheizen der Reste des Lösungsmittels in der festen Phase ( US-A 3 986 269, DE-A 20 53 876).

Granulate erhält man, wenn möglich, durch direktes Abspinnen der Schmelze und anschließender Granulierung oder aber durch Verwendung von Austragsextrudern von denen in Luft oder unter Flüssigkeit, meist Wasser, abgesponnen wird. Werden Extruder benutzt, so kann man der Schmelze, vor diesem Extruder, gegebenenfalls unter Einsatz von statischen Mischern oder durch Seitenextruder im Extruder, Additive zusetzen.

Der Zusatz von Additiven zu Polycarbonat, bzw. zu den Polycarbonat-Verbindungen dient der Verlängerung der Nutzungsdauer oder der Farbe (Stabilisatoren), der Vereinfachung der Verarbeitung (z.B. Entformer, Fließhilfsmittel, Antistatika) oder der Anpassung der Eigenschaften des resultierenden Polycarbonat-Materials an bestimmte Belastungen (Schlagzähmodifikatoren, wie Kautschuke; Flammschutzmittel, Farbmittel, Glasfasern).

Ein dritter Erfindungsgegenstand ist ein Mehrkomponenten-System zur Bisphenol-A Herstellung gemäß vorgenanntem Herstellungsverfahren des ersten Erfindungsgegenstandes, enthaltend

i) ein Volumen gefüllt mit Aceton, welches ein Verfahrensprodukt des Schrittes a) des Verfahrens des ersten Erfindungsgegenstandes ist;

ii) ein Volumen gefüllt mit Phenol, welches ein Verfahrensprodukt des Schrittes b) des Verfahrens des ersten Erfindungsgegenstandes ist;

iii) mindestens ein Reaktor zur Herstellung von Bisphenol-A, welcher mindestens einen Einlass für besagtes Aceton und mindestens einen Einlass für besagtes Phenol enthält, wobei der Einlass für besagtes Aceton in Fluidverbindung mit dem Volumen des besagten Acetons und der Einlass für besagtes Phenol in Fluidverbindung mit dem Volumen des besagten Phenols steht.

Unter einer "Fluidverbindung" wird ein Teil des Mehrkomponenten-Systems verstanden, der andere Teile des Mehrkomponenten-Systems miteinander verbindet und durch den sich ein Stoff, der in jedem Aggregatzustand vorliegen kann, als Stoffstrom von einem Teil zum nächsten transportieren lässt, beispielsweise eine Zuleitung in Form eines Rohres. Der Begriff "in Fluidverbindung stehen" bedeutet, dass benannte Teile des Mehrkomponenten-Systems über eine Fluidverbindung miteinander verbunden sind.

Das "Volumen gefüllt mit Aceton" des Mehrkomponenten-Systems kann beispielsweise das Volumen eines Lagerbehälters oder das Volumen einer Rohrleitung sein. Ist besagtes Volumen ein Lagerbehälter, so ist es im Rahmen einer weiteren Ausführungsform bevorzugt, wenn das Mehrkomponenten-System zusätzlich mindestens eine Entladevorrichtung für bewegliche, abgeschlossene Transportbehälter vorsieht, die in Fluidverbindung mit dem Einlass für besagtes Aceton des Reaktors zur Herstellung von Bisphenol-A steht.

Im Falle der Belieferung wird bevorzugt das Aceton als Verfahrensprodukt im Sinne des besagten Schritts a) des erfindungsgemäßen Verfahrens in einem beweglichen, abgeschlossenem Transportbehälter bereitgestellt, und wird gegebenenfalls daraus zur Zwischenlagerung in Lagerbehälter überführt. Besagter beweglicher, abgeschlossener Transportbehälter kann beispielsweise ein Kessel eines Kesselwaggons, eines Tankschiffs oder eines Tanklastkraftwagens sein. Unter abgeschlossen wird verstanden, dass der besagte Transportbehälter an dessen Vorrichtung zur Befüllung bzw. Entnahme des Transportgutes während des Transports verschlossen ist.

Als Lagerbehälter gilt erfindungsgemäß ein Behälter, der an einen festen Ort gebunden ist, wie beispielsweise ein Tanklager. Lagerbehälter gehören zum unbeweglichen Anlagevermögen oder sind ein Teil davon.

Im Falle der Belieferung wird bevorzugt das Phenol als Verfahrensprodukt im Sinne des besagten Schritts b) des erfindungsgemäßen Verfahrens in einem beweglichen, abgeschlossenem Transportbehälter bereitgestellt, und wird gegebenenfalls daraus zur Zwischenlagerung in Lagerbehälter überführt. Besagter beweglicher, abgeschlossener Transportbehälter kann beispielsweise ein Kessel eines Kesselwaggons, eines Tankschiffs oder eines Tanklastkraftwagens sein. Unter abgeschlossen wird verstanden, dass der besagte Transportbehälter an dessen Vorrichtung zur Befüllung bzw. Entnahme des Transportgutes während des Transports verschlossen ist.

Alle Ausführungsformen des Schritts a) des erfindungsgemäßen Verfahrens zur Herstellung von Bisphenol-A sind auch für das Mehrkomponenten-System *mutatis mutandis* gültig.

Alle Ausführungsformen des Schritts b) des erfindungsgemäßen Verfahrens zur Herstellung von Bisphenol-A sind auch für das Mehrkomponenten-System *mutatis mutandis* gültig.

Ein weiterer Gegenstand ist die Verwendung einer Mischung, enthaltend Aceton als Produkt eines Verfahrens mit mindestens den im ersten Erfindungsgegenstand beschriebenen Schritten a) i) und a) ii) und Phenol als Produkt eines Verfahrens mit mindestens den im ersten Erfindungsgegenstand beschriebenen Schritten b) i) und b) ii) zur Herstellung von Bisphenol-A.

Alle Ausführungsformen des Schritts a) des erfindungsgemäßen Verfahrens zur Herstellung von Bisphenol-A

sind auch für diese Verwendung *mutatis mutandis* gültig.

Alle Ausführungsformen des Schritts b) des erfindungsgemäßen Verfahrens zur Herstellung von Bisphenol-A sind auch für diese Verwendung *mutatis mutandis* gültig.

Die nachfolgenden Beispiele werden exemplarisch zur Verdeutlichung der Erfindung angeführt, ohne die Erfindung auf den Offenbarungsgehalt der Beispiele zu beschränken.

**Beispiele**

In den Fig. 2 und 3 werden beispielhafte Ausführungsformen des erfindungsgemäßen Verfahrens dargestellt.

**Beispiel 1:**

In Fig.2 wird eine Ausführungsform des erfindungsgemäßen Verfahrens illustriert. Darin haben die Bezugszeichen die nachfolgende Bedeutung:

A1 = $CO_2$
B1 = Wasserstoffgas als Produkt einer Wasserelektrolyse
C = Produktgasgemisch enthaltend CO
D = Synthesegas
E = Propen
F = Bisphenol-A
G1 = Plastikabfall als polymere, organische Verbindungen
H1 = Benzol als Vorläufer gemäß Formel (I), wenn nicht $R^1$ = Hydroxylgruppe und $R^2$ = Wasserstoffatom
H2 = Cumolhydroperoxid als oxidierte, aromatische Verbindung
I = Phenol
J = gasförmiges Pyrolysat
1a = CO Bereitstellung durch RWGS
2 = Umsetzung von CO und Wasserstoffgas **B1** zu Methanol
3 = Bereitstellung von Methanol aus **2**
4 = Umsetzung des Methanols zu Propen mittels MTP-Prozess
5 = Oxidation des Propens **E** zu Aceton
6 = Bereitstellung von Aceton
7 = Synthese von Bisphenol-A unter Einsatz von bereitgestelltem Aceton und bereitgestelltem Phenol
8 = Bereitstellung von Phenol
9 = Umwandlung des Ausgangsmaterials **G1** zu Benzol als einem Vorläufer **H1**
9a = Pyrolyse zur Umwandlung des Ausgangsmaterials **G1** zu gasförmigem Pyrolysat
9b = katalytische Konversion des gasförmigen Pyrolysates zu
10a = Umwandlung des Vorläufers aus **9a** zu Cumolhydroperoxid
10b = Umwandlung des Cumolhydroperoxids in Aceton und Phenol
11 = Elektrolyse von Wasser unter Einsatz von elektrischem Strom aus regenerativer Energie

Die Synthese **7** von Bisphenol-A **F** wird nach der Bereitstellung von Aceton 6 durchgeführt. Das bereitgestellte Aceton ist hierbei zwingend ein Produkt eines Verfahrens, in welchem das Aceton über bereitgestelltes Methanol **3** als Zwischenprodukt hergestellt wird. Dabei wird zwingend zumindest Kohlenmonoxid in einem Kohlenmonoxid enthaltenden Produktgasstroms **C** in einer reverse-watergas-shift Reaktion **1a** (RWGS) durch Reduktion von Kohlendioxid **A1** mit aus einer Wasserelektrolyse **11** erhaltenem Wasserstoffgas **B1** erhalten und das nach Durchlauf einer in einer Prozessgasaufbereitung (Punkt in Fig.2) stattfindenden Aufreinigung des Kohlenmonoxids aus dem Produktgasstrom **C** und Zusatz von Wasserstoffgas **B1** erhaltene Synthesegas **D** zu Methanol umgesetzt und dieses Methanol weiter in einem MTP-Prozess **4** zu Propen **E** umgesetzt, wobei das Propen **E** zur Oxidation **5** des Propens zunächst mit Benzol als Vorläufer **H1** zu Cumol umgewandelt und der resultierende Propan-2-yl-Rest des Cumols nach Oxidation in 2-Position zu einem 2-Hydroperoxy-propan-2-yl-Rest des Cumolhydroperoxid **H2** umgesetzt **10a** und danach mittels oxidativer Abspaltung zu Aceton umgewandelt **10b** wird.

Die Synthese **7** von Bisphenol-A **F** wird ebenso nach der Bereitstellung von Phenol **8** durchgeführt. Das bereitgestellte Phenol ist hierbei zwingend ein Produkt eines Verfahrens, in dem das Phenol aus Benzol als Vorläufer **H1** hergestellt wird, wobei das Benzol wiederum ein Produkt einer katalytischen Konversion **9b** von gasförmigem Pyrolysat **J** einer Pyrolyse **9a** von polymerer, organischer Verbindung **G1** in Form von Plastikabfall ist. Das Benzol wird für das bereitgestellte Phenol in einer Oxidation **5** von Propen zunächst wie oben beschrieben zu Cumolhydroperoxid umgesetzt **10a,** und in der oxidativen Abspaltung **10b** zu Phenol umgewandelt.

**Beispiel 2:**

In Fig.3 wird eine Ausführungsform des erfindungsgemäßen Verfahrens illustriert. Darin haben die Bezugszeichen die Bedeutung wie in Beispiel 1, sofern nicht nachfolgend geändert:

A1 = Plastikabfall als organische, festförmige Verbindung
B2 = Sauerstoff-haltiges Gas (z.B. $O_2$ als Produkt der Wasserelektrolyse **11**)
1b = CO Bereitstellung durch partielle Oxidation

Die Synthese **7** von Bisphenol-A **F** wird nach der Bereitstellung von Aceton **6** durchgeführt. Das bereitgestellte Aceton ist hierbei zwingend ein Produkt eines Verfahrens, in welchem das Aceton über bereitgestelltes Methanol **3** als Zwischenprodukt hergestellt wird. Dabei wird zwingend zumindest Kohlenmonoxid in einem Kohlenmonoxid enthaltenden Produktgasstroms **C** durch eine partiellen Oxidation **1b** von Plastikabfall **A1** mit aus einer Wasserelektrolyse **11** erhaltenem Sauerstoffgas **B2** erzeugt und das nach Durchlauf einer in einer Prozessgasaufbereitung (Punkt in Fig.2) stattfindenden Aufreinigung des Kohlenmonoxids des Produktgasstromes **C** und Zusatz von Wasserstoffgas **B1** erhaltene Synthesegas **D** zu Methanol umgesetzt und dieses Methanol weiter in einem MTP-Prozess **4** zu Propen **E** umgesetzt, wobei das Propen **E** zur Oxidation **5** des Propens zunächst mit Benzol als Vorläufer **H1** zu Cumol umgewandelt und der resultierende Propan-2-yl-Rest des Cumols nach Oxidation in 2-Position zu einem 2-Hydroperoxy-propan-2-yl-Rest des Cumolhydroperoxid **H2** umgesetzt **10a** und danach mittels oxidativer Abspaltung zu Aceton umgewandelt **10b** wird.

Die Synthese **7** von Bisphenol-A **F** wird ebenso nach der Bereitstellung von Phenol **8** durchgeführt. Das bereitgestellte Phenol ist hierbei zwingend ein Produkt eines Verfahrens, in dem das Phenol aus Benzol als Vorläufer **H1** hergestellt wird, wobei das Benzol wiederum ein Produkt einer katalytischen Konversion **9b** von gasförmigem Pyrolysat **J** einer Pyrolyse **9a** von polymerer, organischer Verbindung **G1** in Form von Plastikabfall ist. Das Benzol wird für das bereitgestellte Phenol in einer Oxidation **5** von Propen zunächst wie oben beschrieben zu Cumolhydroperoxid umgesetzt **10a,** und in der oxidativen Abspaltung **10b** zu Phenol umgewandelt.

**Patentansprüche**

**1.** Verfahren zur Herstellung von Bisphenol-A (F) für die Herstellung von Polycarbonat, enthaltend zumindest die Schritte:

a) Bereitstellung (4) von Aceton, welches ein Verfahrensprodukt eines Verfahrens enthaltend mindestens folgende Schritte ist:

i) Bereitstellung (3) von Methanol, welches ein Verfahrensprodukt eines Verfahrens mit mindestens folgenden Schritten ist:
i-1) Umsetzung (2) von CO zu Methanol, wobei das CO ein Verfahrensprodukt mindestens einer partiellen Reduktion (1) von $CO_2$ zu CO und/oder ein Verfahrensprodukt mindestens einer partiellen Oxidation (1) von organischem Material zu CO ist, wobei mindestens ein organisches Material (A) ausgewählt wird aus mindestens einer organischen, festförmigen Verbindung, Methan aus biologischer Quelle oder Mischungen daraus;
ii) Synthese von Aceton aus zuvor bereitgestelltem Methanol (3) durch zumindest folgende Schritte:

Bereitstellung (4) von Propen als Produkt eines Verfahrens mit zumindest folgenden Schritten:

ii-1) Umsetzung des zuvor bereitgestellten Methanols (3) zu einer Produktmischung, enthaltend Dimethylether, Wasser und Methanol;
ii-2) Umwandlung von Ausgangsmaterial, enthaltend Dimethylether, Wasser und Methanol jeweils aus der vorgenannten Produktmischung, bei einer Temperatur von mehr als 200°C durch Kontakt mit einem Katalysator, vorzugsweise mit mindestens einer Zeolith-Verbindung als Katalysator, zu Propen (E);

Umsetzung des Propens (E) durch zumindest
ii-3) Oxidation (5) des Propens (E) zu Aceton;

b) Bereitstellung (8) von Phenol, welches ein Verfahrensprodukt mindestens eines Herstellverfahrens ist, das zumindest folgende Schritte enthält:

i) Bereitstellung mindestens einer organischen, aromatischen Verbindung gemäß Formel (I),

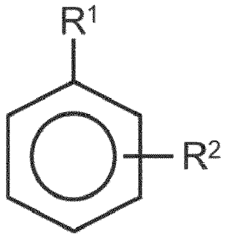

(I)

worin

$R^1$ für ein Wasserstoffatom, eine Hydroxylgruppe, Methyl, Carboxyl, Carboxylat oder $-CHR^3R^4$ steht, worin $R^3$ und $R^4$ unabhängig voneinander für eine $(C_1-C_3)$-Alkylgruppe stehen oder gemeinsam mit dem Restmolekül einen aliphatischen Kohlenwasserstoffzyklus bilden; und

$R^2$ für ein Wasserstoffatom, Methyl, Carboxyl oder Carboxylat steht, mit der Maßgabe, dass falls $R^1$ für $-CHR^3R^4$ steht, $R^2$ ein Wasserstoffatom bedeutet und falls $R^1$ für eine Hydroxylgruppe steht, $R^2$ für ein Wasserstoffatom, Carboxyl oder Carboxylat steht,

wobei die besagte organische, aromatische Verbindung Verfahrensprodukt einer Umwandlung (9) mindestens eines Ausgangsstoffes ausgewählt aus mindestens einer Verbindung aus der Gruppe, die gebildet wird aus polymeren organischen Verbindungen, Kohlehydraten und Methan aus biologischer Quelle, ist;

und falls in besagter organischer, aromatischer Verbindung aus Schritt i) gemäß Formel (I) $R^1$ nicht für eine Hydroxylgruppe steht und $R^2$ nicht für ein Wasserstoffatom steht

ii) mindestens einen der Schritte ii-1) und/oder ii-2) und/oder iii-3)

ii-1) Umsetzung der organischen, aromatischen Verbindung (H) aus Schritt i) mit Propen unter Erhalt einer Propan-2-yl substituierten aromatischen, organischen Verbindung als Produkt;

ii-2) Oxidation der organischen, aromatischen Verbindung (H) aus Schritt i) und/oder des Produkts aus Schritt ii-1), wobei mindestens eine oxidierte, aromatische, organische Verbindung erhalten wird;

ii-3) Abspaltung mindestens eines kohlenstoffhaltigen Substituenten der organischen, aromatischen Verbindung (H) aus Schritt i) und/oder der oxidierten, organischen, aromatischen Verbindung aus der Oxidation gemäß Schritt ii-2);

c) Umsetzung (7) einer Mischung, enthaltend das bereitgestellte Aceton und das bereitgestellte Phenol, zu Bisphenol-A (F).

**2.** Verfahren gemäß Anspruch 1, **dadurch gekennzeichnet, dass** das bei der Umsetzung (2) gemäß Schritt a) i-1) eingesetzte CO ein Verfahrensprodukt mindestens einer partiellen Reduktion eines Gasstroms ist, der mindestens $CO_x$ mit x = 1 oder 2 und gegebenenfalls Wasserstoffgas (B) enthält.

**3.** Verfahren gemäß einem der Ansprüche 1 oder 2, **dadurch gekennzeichnet, dass** das CO in Schritt a) i-1) ein Verfahrensprodukt mindestens einer partiellen Reduktion von $CO_2$ zu CO ist, insbesondere ausgewählt aus einer reverse-watergas-shift Reaktion unter Einsatz von mittels Elektrolyse bereitgestelltem $H_2$ (B), aus einer elektrochemischen Reduktion von $CO_2$ zu CO oder aus Mischungen daraus.

**4.** Verfahren gemäß einem der vorangehenden Ansprüche, **dadurch gekennzeichnet, dass** das CO in Schritt a) i-1) als Verfahrensprodukt mindestens einer reverse-watergas-shift Reaktion unter Einsatz von mittels Elektrolyse bereitgestelltem $H_2$ (B) ist oder aus einer elektrochemischen Reduktion von $CO_2$ zu CO oder aus Mischungen daraus bereitgestellt wird, mit der Maßgabe, dass hierbei aus regenerativer Energie hergestellte elektrischer Energie genutzt wird.

**5.** Verfahren gemäß einem der vorangehenden Ansprüche, **dadurch gekennzeichnet, dass** die Umsetzung des Methanols gemäß Schritt a) ii-1) durch Kontaktieren des Methanols, bevorzugt des Methanols in der Gasphase, mit einem Katalysator erfolgt.

**6.** Verfahren gemäß einem der vorangehenden Ansprüche, **dadurch gekennzeichnet, dass** zur Umsetzung des Methanols in Schritt a) ii-1) das besagte bereitgestellte Methanol eine Temperatur in einem Bereich von 200 °C bis 350°C aufweist.

**7.** Verfahren gemäß einem der vorangehenden Ansprüche, **dadurch gekennzeichnet, dass** die Umwandlung des Ausgangsmaterials gemäß Schritt a) ii-2) bei einer Temperatur von 350°C bis 600°C, insbesondere von 380°C bis 550°C, erfolgt.

**8.** Verfahren gemäß einem der vorangehenden Ansprüche, **dadurch gekennzeichnet, dass** im Schritt a) ii-3) die Oxidation (5) des Propens (E) nach mindestens einer der folgenden Methoden durchgeführt wird:

1) gemäß Wacker-Hoechst-Prozess durch Umsetzung von Propen (E) und Wasser an einem Katalysatorsystem aus Palladium(II)chlorid und Kupfer(II)chlorid zu Aceton;
2) Hydroxylierung von Propen (E) mit Wasser zu 2-Propanol und anschließender katalytischer Dehydrogenierung von 2-Propanol zu Aceton;
3) Umsetzung des Propens (E) zu Cumol und Durchführung des Cumolhydroperoxid-Verfahrens oder Umsetzung des Propens (E) zu Diisopropylbenzol und Durchführung des Cumolhydroperoxidverfahrens mit Diisopropylbenzolhydroperoxid anstelle von Cumolhydroperoxid, jeweils unter Erhalt von Aceton.

**9.** Verfahren gemäß einem der vorangehenden Ansprüche, **dadurch gekennzeichnet, dass** gemäß Formel (I) die Reste $R^3$ und $R^4$ unabhängig voneinander ausgewählt sind aus Methyl, Ethyl, Propyl oder gemeinsam mit dem Restmolekül einen 6-gliedrigen aliphatischen Kohlenwasserstoffring, insbesondere Cyclohexyl, bilden.

**10.** Verfahren gemäß einem der vorangehenden Ansprüche, **dadurch gekennzeichnet, dass** gemäß Formel (I) der Rest $-CHR^3R^4$ ausgewählt wird aus Propan-2-yl, sec-Butyl oder Cyclohexyl.

**11.** Verfahren gemäß einem der vorangehenden Ansprüche, **dadurch gekennzeichnet, dass** als Ausgangsstoff des Schrittes b) i) mindestens eine Verbindung ausgewählt wird aus der Gruppe, die gebildet wird aus Polysachariden, Trisacchariden, C-6 Monosacchariden, C-5 Monosacchariden, Methan aus biologischer Quelle und homopolymeren und copolymeren Verbindungen aus der Gruppe Cellulose, Lignin, Polyester, Polyamid, Polyurethan (PUR), Polyharnstoff, Polyisocyanurat (PIR), Polycarbonat.

**12.** Verfahren gemäß einem der vorangehenden Ansprüche **dadurch gekennzeichnet, dass** in Schritt b) i) Benzol als organische, aromatische Verbindung bereitgestellt wird und das Benzol in die Oxidation (5) von Propen (E) in Schritt a) ii-3) eingebracht wird und dort mit dem aus Schritt a) ii-2) bereitgestellten Propen (E) zu Cumol umgesetzt wird und das gebildete Cumol anschließend oxidiert wird unter Bereitstellung von Phenol und Aceton.

**13.** Verfahren gemäß einem der vorangehenden Ansprüche, **dadurch gekennzeichnet, dass** in Schritt b) i) die organische, aromatische Verbindung, insbesondere Toluol und/oder Benzol, durch Pyrolyse des Ausgangsstoffes bei einer Temperatur von 400 bis 1000°C bereitgestellt wird.

**14.** Verfahren zur Herstellung von Polycarbonat, enthaltend zumindest die folgenden Schritte:

Herstellung von Bisphenol-A, welches ein Verfahrensprodukt des Verfahrens nach einem der Ansprüche 1 bis 13 ist;
Herstellung mindestens eines Kohlensäurederivats, ausgewählt aus Phosgen, Kohlensäurediester oder Mischungen daraus;
Umsetzung des besagten Kohlensäurederivats mit mindestens einem Diphenol, mit der Maßgabe, dass zumindest das zuvor hergestellte Bisphenol-A als Diphenol eingesetzt wird.

**15.** Verfahren nach Anspruch 14, **dadurch gekennzeichnet, dass** zur Herstellung des Kohlensäurederivates Kohlenmonoxid als Ausgangsstoff bereitgestellt wird, welches ein Verfahrensprodukt mindestens eines der folgenden Verfahren ist, ausgewählt aus einer RWGS unter Einsatz von $H_2$ als Verfahrensprodukt einer Wasserelektrolyse, aus einer Gasification, aus einer elektrochemischen Reduktion von $CO_2$ zu CO oder aus Mischungen daraus.

**16.** Mehrkomponenten-System zur Bisphenol-A Herstellung nach einem Herstellungsverfahren der Ansprüche 1 bis 13, enthaltend

i) ein Volumen gefüllt mit Aceton, welches ein Verfahrensprodukt des Schrittes a) des Verfahrens gemäß einem der Ansprüche 1 bis 13 ist;
ii) ein Volumen gefüllt mit Phenol, welches ein Verfahrensprodukt des Schrittes b) des Verfahrens gemäß einem der Ansprüche 1 bis 13 ist;
iii) mindestens ein Reaktor zur Herstellung von Bisphenol-A, welcher mindestens einen Einlass für besagtes Aceton und mindestens einen Einlass für besagtes Phenol enthält, wobei der Einlass für besagtes Aceton in Fluidverbindung mit dem Volumen des besagten Acetons und der Einlass für besagtes Phenol in Fluidverbindung mit dem Volumen des besagten Phenols steht.

Fig.1:

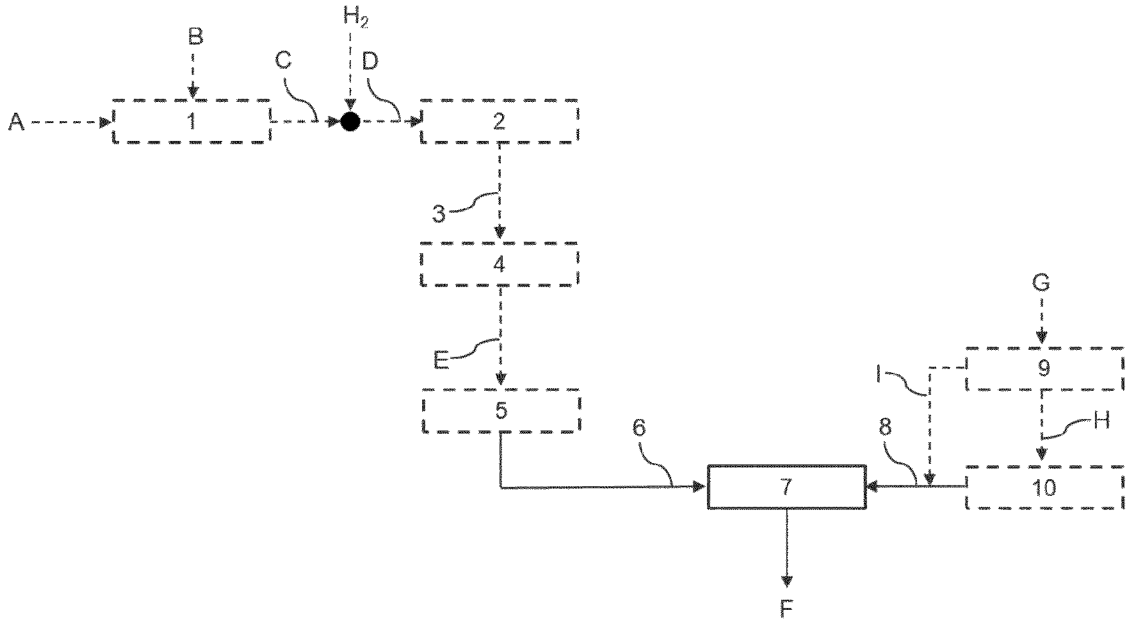
B
H2
C
D
A
1
2
3
4
G
E
I
9
5
H
6
8
7
10
F

Fig.2:

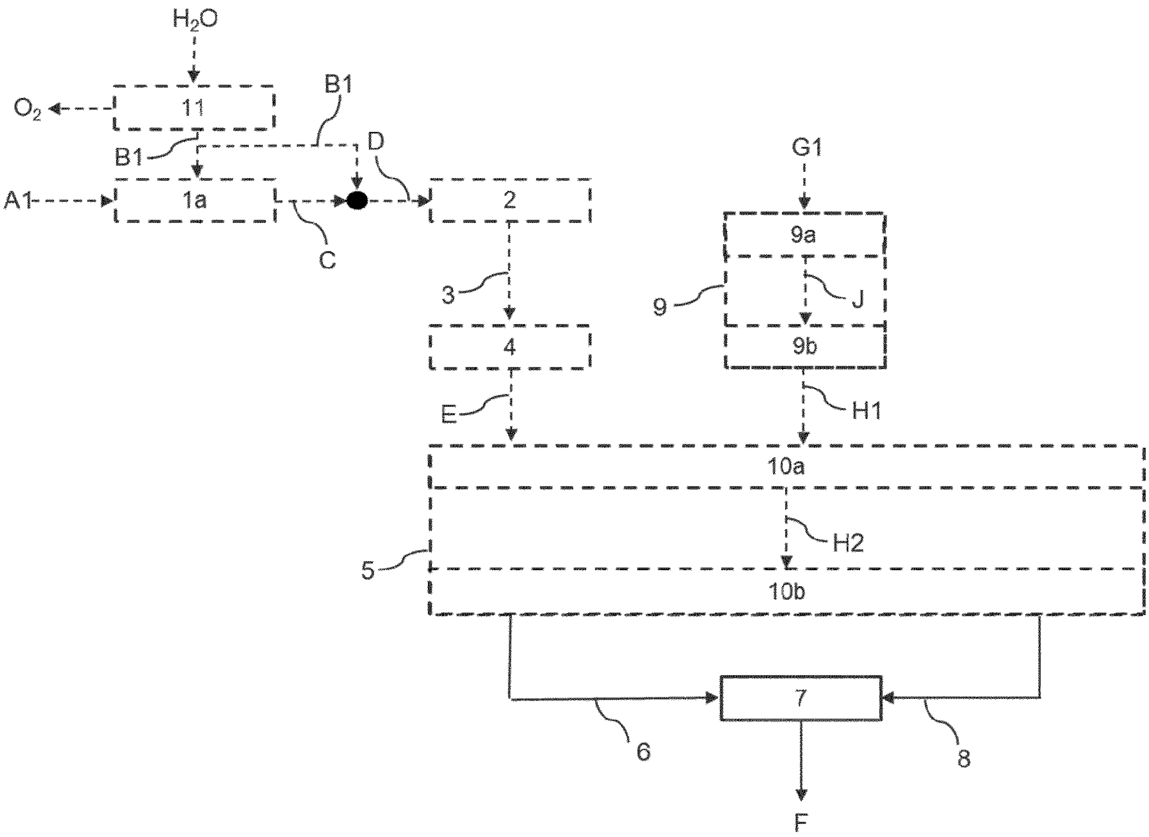
H2O
B1
O2
11
B1
D
G1
A1
1a
2
C
9a
3
9
J
4
9b
E
H1
10a
H2
5
10b
7
6
8
F

Fig.3:

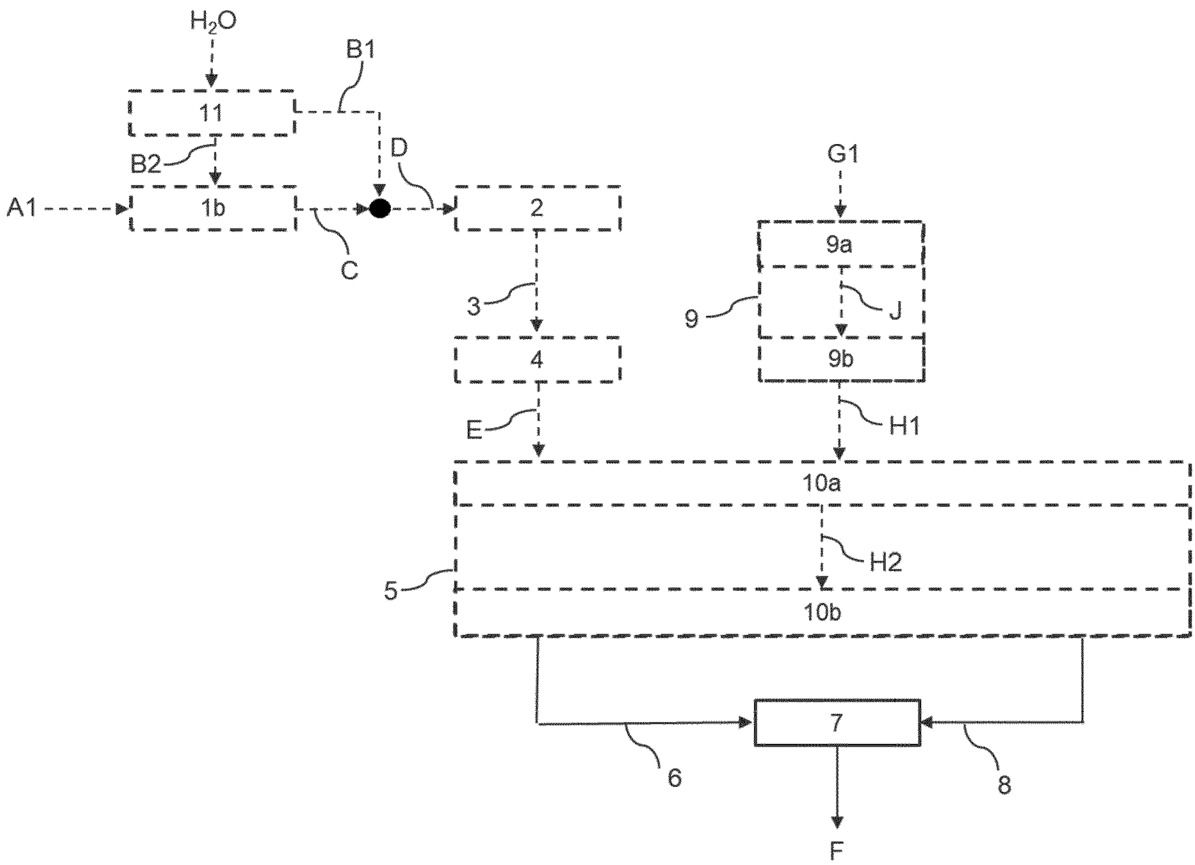

Europäisches Patentamt
European Patent Office
Office européen des brevets

# EUROPÄISCHER RECHERCHENBERICHT

Nummer der Anmeldung
EP 23 16 5364

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X<br>A | US 2010/152406 A1 (MAHOOD JAMES A [US]) 17. Juni 2010 (2010-06-17)<br>* Absätze [0004], [0006], [0055], [0063] - [0066], [0071] - [0072] * | 16<br>1-15 | INV.<br>C07C37/20<br>C07C39/16<br>C07C29/151<br>C07C1/20<br>C07C11/06<br>C07C41/09<br>C07C43/04<br>C07C45/28<br>C07C49/08 |
| A | US 2011/172475 A1 (PETERS MATTHEW W [US] ET AL) 14. Juli 2011 (2011-07-14)<br>* Absätze [0134], [0139]; Ansprüche 1,18,20 * | 1-15 | |
| A | US 7 205 376 B2 (GEN ELECTRIC [US]) 17. April 2007 (2007-04-17)<br>* Ansprüche 1,9 * | 1-15 | |
| A | Manfred Weber ET AL: "Phenol" In: "Ullmann's Encyclopedia of Industrial Chemistry", 23. März 2020 (2020-03-23), Wiley-VCH, Weinheim, XP055694463, ISBN: 978-3-527-30673-2 Seiten 1-20, DOI: 10.1002/14356007.a19_299.pub3,<br>* section 4.5.4 * | 1-13 | |
| | | | RECHERCHIERTE SACHGEBIETE (IPC) |
| | | | C07C |

Der vorliegende Recherchenbericht wurde für alle Patentansprüche erstellt

1

| Recherchenort | Abschlußdatum der Recherche | Prüfer |
|---|---|---|
| Den Haag | 21. September 2023 | Matés Valdivielso, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 23 16 5364

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

21-09-2023

| Im Recherchenbericht angeführtes Patentdokument | Datum der Veröffentlichung | Mitglied(er) der Patentfamilie | Datum der Veröffentlichung |
|---|---|---|---|
| US 2010152406 A1 | 17-06-2010 | KEINE | |
| US 2011172475 A1 | 14-07-2011 | BR 112012016883 A2 | 05-06-2018 |
| | | CA 2786607 A1 | 14-07-2011 |
| | | CN 102770397 A | 07-11-2012 |
| | | EP 2521705 A1 | 14-11-2012 |
| | | JP 2013516487 A | 13-05-2013 |
| | | MY 159813 A | 15-02-2017 |
| | | SG 182407 A1 | 30-08-2012 |
| | | US 2011172475 A1 | 14-07-2011 |
| | | US 2013261323 A1 | 03-10-2013 |
| | | WO 2011085223 A1 | 14-07-2011 |
| US 7205376 B2 | 17-04-2007 | EP 1748973 A1 | 07-02-2007 |
| | | US 2006020047 A1 | 26-01-2006 |
| | | WO 2005110952 A1 | 24-11-2005 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 20100152406 A1
- EP 2022052267 W
- WO 2021089737 A
- WO 2021069470 A
- WO 2020057998 A1
- EP 0448000 B1
- DE 19723363 A1
- US 7015369 B2
- WO 2020204707 A1
- US 9328297 B1
- WO 2006068814 A
- WO 2014076113 A1
- WO 0035847 A
- EP 1728777 A1
- US 2775620 A
- EP 0881986 A1
- EP 1640341 A2
- DE 3327274 A1
- GB 583477 A
- WO 9730932 A1
- WO 9616898 A1
- US 6713035 B1
- DE 102009011874 A1
- EP 0304691 A2
- BE 866991 A
- EP 0411510 A
- US 4980105 A
- DE 3332065 A
- EP 0267025 A
- EP 0460450 A
- EP 003996 A
- EP 0256003 A
- US 4423207 A
- DE 3429960 A
- US 3986269 A
- DE 2053876 A


### In der Beschreibung aufgeführte Nicht-Patentliteratur


- **DITTMEYER et al.** *Chemische Technik,* vol. 4
- **VOLKMAR M. SCHMIDT.** Elektrochemische Verfahrenstechnik. Wiley-VCH-Verlag, 2003
- **JÜRGEN CARL.** Noell-Konversionsverfahren zur Verwertung und Entsorgung von Abfällen. EF-Verl. für Energie- und Umwelttechnik, 1994
- **OTT et al.** Methanol in: Ullmann's Encyclopedia of industrial chemistry. Wiley-VCH Verlag, 2012
- **WEBER et al.** Acetone in: Ullmann's Encyclopedia of industrial chemistry. Wiley-VCH Verlag, 2013
- **B. COOK et al.** *Applied Catalysis A: General,* 2009, vol. 365, 34-41
- **ZURBEL et al.** *Chem. Ing. Tech.,* 2019, vol. 4, 484-493
- **HUANG et al.** *ACS Catal.,* 2018, vol. 8
- Polycarbonates. **V. SERINI.** Ullmann's Encyclopedia of Industrial Chemistry. Wiley-VCH, 2000
- Thermische Trennverfahren. VCH Verlagsanstalt, 1988, 114
- **VAUCK.** Grundoperationen chemischer Verfahrenstechnik. Deutscher Verlag für Grundstoffindustrie, 2000, 690